(19) Europäisches Patentamt
European Patent Office
Office européen des brevets

(11) Publication number: **0 336 369 A1**

(12) **EUROPEAN PATENT APPLICATION**

(21) Application number: 89105883.6

(22) Date of filing: 04.04.89

(51) Int. Cl.⁴: C07D 401/14 , C07D 401/06 , C07D 213/69 , A61K 31/44 , A61K 31/495 , A61K 31/505

(30) Priority: 04.04.88 US 177207

(43) Date of publication of application: 11.10.89 Bulletin 89/41

(84) Designated Contracting States: AT BE CH DE ES FR GB GR IT LI LU NL SE

(71) Applicant: E.R. Squibb & Sons, Inc.
Lawrenceville-Princeton Road
Princeton, N.J. 08540(US)

(72) Inventor: Treuner, Uwe D.
Am Goldberg Sonnenstrasse 6
Etterzhausen(DE)

(74) Representative: Vossius & Partner
Siebertstrasse 4 P.O. Box 86 07 67
D-8000 München 86(DE)

(54) **3-Acylamino-1-[[[(substituted sulfonyl)amino]carbonyl]amino]2-azetidinones.**

(57) Compounds having the formula

I

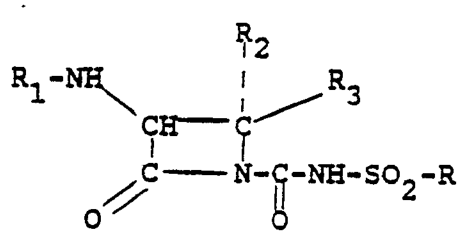

,

and pharmaceutically acceptable salts thereof, exhibit antibacterial activity.

EP 0 336 369 A1

R is 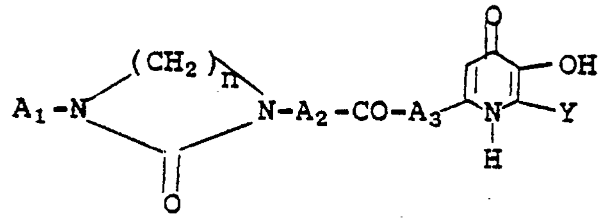

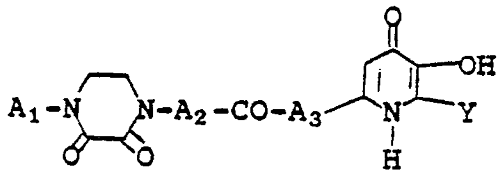

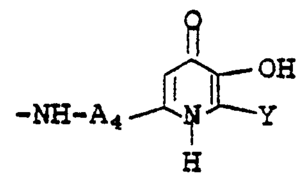

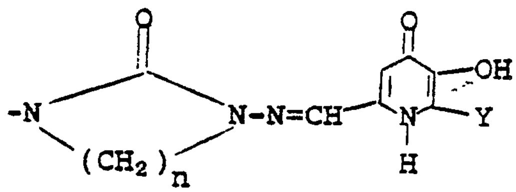

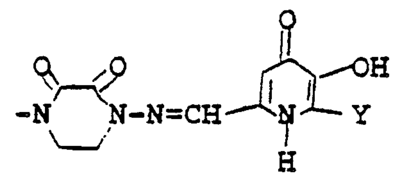

# 3-ACYLAMINO-1-[[[(SUBSTITUTED SULFONYL) AMINO]CARBONYL]AMINO] 2-AZETIDINONES

Compounds having the formula

I

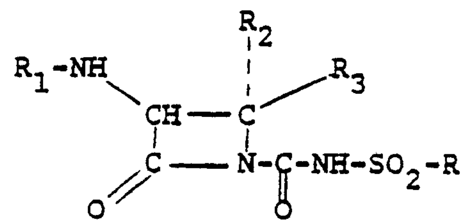

,

and pharmaceutically acceptable salts thereof, exhibit antibacterial activity. In formula I, and throughout the specification, the symbols are as defined below.

R is 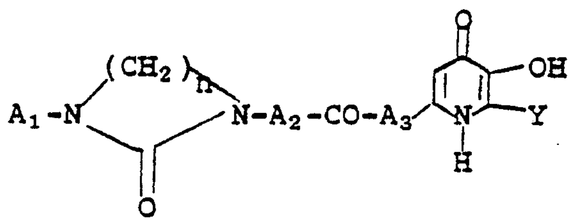

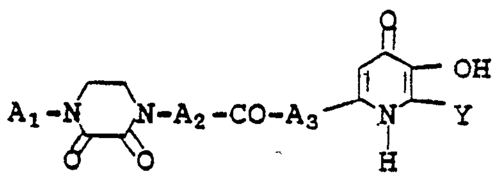

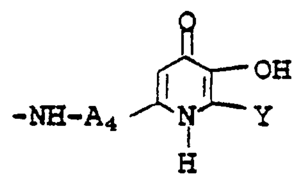

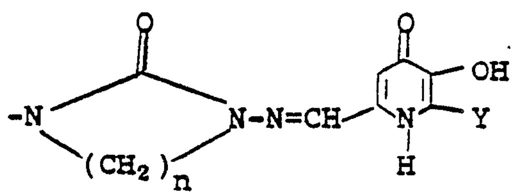

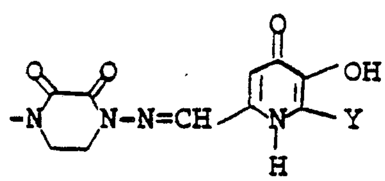

$R_1$ is an acyl group derived from a carboxylic acid;

$R_2$ and $R_3$ are the same or different and each is hydrogen, alkyl, alkenyl, alkynyl, cycloalkyl, phenyl, substituted phenyl or a 4, 5, 6 or 7-membered herterocycle (hereinafter referred to as $R_x$), or one of $R_2$ and $R_3$ is hydrogen and the other is azido, halomethyl, dihalomethyl, trihalomethyl, alkoxycarbonyl, 2-phenylethenyl, 2-phenylethynyl, carboxyl, $-CH_2X_1$ [wherein $X_1$ is azido, amino ($-NH_2$), hydroxy, carboxyl, alkoxycarbonyl, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano, $-A-\overset{O}{\overset{\|}{C}}-NX_6X_7$, $-S-X_2$, or $-O-X_2$ (wherein A, $X_2$, $X_6$ and $X_7$ are as hereinafter defined)], $-S-X_2$ or $-O-X_2$ - [wherein $X_2$ is alkyl, substituted alkyl, phenyl, substituted phenyl, phenylalkyl, (substituted phenyl)alkyl, alkanoyl, substituted alkanoyl, phenylalkanoyl, (substituted phenyl)-alkanoyl, phenylcarbonyl, (substituted phenyl)carbonyl, or heteroarylcarbonyl],

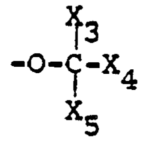 or

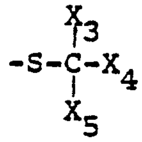

[wherein one of $X_3$ and $X_4$ is hydrogen and the other is hydrogen or alkyl, or $X_3$ and $X_4$ when taken together with the carbon atom to which they are attached form a cycloalkyl group; and $X_5$ is formyl, alkanoyl, phenylcarbonyl, (substituted phenyl)carbonyl, phenylalkylcarbonyl, (substituted phenyl)-alkylcarbonyl, carboxyl, alkoxycarbonyl, aminocarbonyl

($NH_2-\overset{O}{\overset{\|}{C}}-$), (substituted amino)carbonyl, or cyano (-C≡N)], or

$A-\overset{O}{\overset{\|}{C}}-NX_6X_7$ [wherein A is -CH=CH-, $-(CH_2)_m-$, $-(CH_2)_m-O-$, $-(CH_2)_m-NH-$, or $-CH_2-S-CH_2-$, m is 0, 1 or 2, and $X_6$ and $X_7$ are the same or different and each is hydrogen, alkyl, phenyl or substituted phenyl, or $X_6$ is hydrogen and $X_7$ is amino, substituted amino, alkanoylamino or alkoxy, or $X_6$ and $X_7$ when taken together with the nitrogen atom to which they are attached form a 4, 5, 6 or 7-membered heterocycle];

$A_1$ is a single bond, NH or $NH-NH-\overset{O}{\overset{\|}{C}}$;

$A_2$ is a single bond, NH, $CH_2-CH_2-NH$ or $\overset{O}{\overset{\|}{C}}NH-NH$;

$A_3$ is a single bond, CH=CH, $(CH_2)_2-$;

$A_4$ is $NH-\overset{O}{\overset{\|}{C}}-$; $\overset{O}{\overset{\|}{C}}-CH=CH-$; $\overset{O}{\overset{\|}{C}}-(CH_2)_2-$;

n is two or three

Y is $CH_2X$, $CHF_2$, $\overset{O}{\overset{\|}{C}}-H$, C≡N, $\overset{O}{\overset{\|}{C}}-OR_4$, 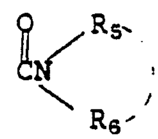, $OR_7$, $CH=NR_8$, $CH=CHR_9$, $\overset{O}{\overset{\|}{C}}NHOR_{10}$, or $\underset{S}{\underset{\|}{C}}-NHR_{11}$

X is hydrogen, halogen, OH, $OR_{12}$, $NH_2$, $NHR_{13}$, $N_3$, $\overset{(O)m}{\overset{\|}{S}}R_{14}$, C≡N, S-C≡N, $\underset{O}{\underset{\|}{C}}-OR_{15}$, or $\overset{O}{\overset{\|}{C}}NHR_{16}$, 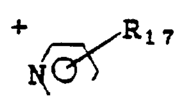

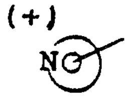 (6 or 5 membered heterocycles containing one or two nitrogen atoms)

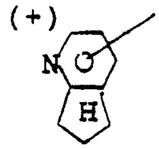 ; 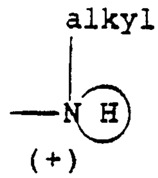 (saturated 5 or 6 membered)

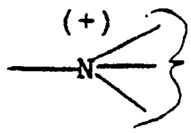 lower alkyl; 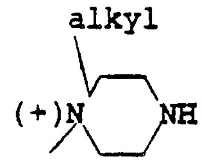

$R_4$ is hydrogen, salt forming metal or ion, lower alkyl;
$R_5$ and $R_6$ is hydrogen or lower alkyl or joined together to form a five or six membered ring;
$R_7$ is hydrogen, lower alkyl, aralkyl;
$R_8$ is O-lower alkyl, hydroxy, NH-alkyl, NH-aryl;
$R_9$ is C≡N, COOH, COO-lower alkyl, $CONH_2$, O-lower alkyl, S-lower alkyl;
$R_{10}$ is hydrogen, lower alkyl;
$R_{11}$ is hydrogen, lower alkyl;
$R_{12}$ is hydrogen, lower alkyl, aralkyl,

$\overset{O}{\overset{\|}{C}}NH_2$, $\overset{O}{\overset{\|}{C}}$NH-lower alkyl, $\overset{O}{\overset{\|}{C}}$NH-aryl;

$R_{13}$ is lower alkyl, $\overset{O}{\overset{\|}{C}}NH_2$, $\overset{O}{\overset{\|}{C}}$NH-lower alkyl, $\overset{O}{\overset{\|}{C}}$NH-aryl, $\overset{O}{\overset{\|}{C}}$NH-heterocycle, $\underset{S}{\underset{\|}{C}}NH_2$, $\underset{S}{\underset{\|}{C}}$NH-lower alkyl, $\underset{S}{\underset{\|}{C}}$NH-aryl;

$R_{14}$ is lower alkyl, m is zero, one, or two;
$R_{15}$ is hydrogen, salt forming metal ion, lower alkyl;
$R_{16}$ is hydrogen, lower alkyl;
$R_{17}$ is hydrogen, $CONH_2$, COOH.

The above symbols are used to represent groups of multiple atoms.

These groups are inserted in the structural formulas shown herein in the order in which they are presented (i.e., from left to right). For

example, if R is 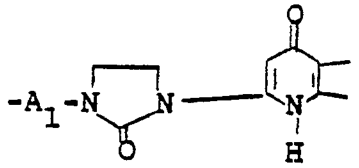 and $A_1$ is

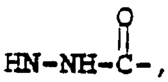, the R group would be 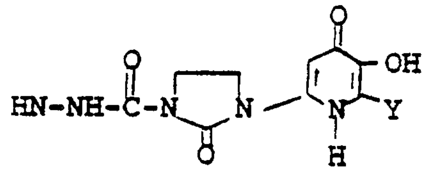

not 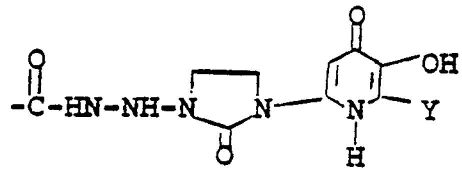.

Listed below are definitions of various terms used to describe the β-lactams of this invention. These definitions apply to the terms as they are used throughout the specification (unless they are otherwise limited in specific instances) either individually or as part of a larger group.

The terms "alkyl" and "alkoxy" refer to both straight and branched chain groups. Those groups having 1 to 10 carbon atoms are preferred.

The terms "cycloalkyl" and "cycloalkenyl" refer to cycloalkyl and cycloalkenyl groups having 3,4,5,6 or 7 carbon atoms.

The term "substituted alkyl" refers to alkyl groups substituted with one or more (preferably 1, 2 or 3) azido, amino ($-NH_2$), halogen, hydroxy, carboxy, cyano, alkoxycarbonyl, aminocarbonyl, alkanoyloxy, alkoxy, phenyloxy, (substituted phenyl)oxy, mercapto, alkylthio, phenylthio, (substituted phenyl)thio, alkylsulfinyl, or alkylsulfonyl groups.

The terms "substituted alkanoyl refers to alkanoyl groups substituted with one or more (preferably 1, 2 or 3 azido, amino ($-NH_2$), halogen, hydroxy, carboxy, cyano, alkoxycarbonyl, aminocarbonyl, alkanoyloxy, alkoxy, phenyloxy, (substituted phenyl)oxy, mercapto, alkylthio, phenylthio, (substituted phenyl)thio, alkylsulfinyl, or alkylsulfonyl groups.

The terms "alkanoyl", "alkenyl", and "alkynyl" refer to both straight and branched chain groups. Those groups having 2 to 10 carbon atoms are preferred.

The terms "halogen" and "halo" refer to fluorine, chlorine, bromine and iodine.

The term "substituted phenyl" refers to a phenyl group substituted with 1, 2 or 3 amino ($-NH_2$), halogen, hydroxyl, trifluoromethyl, alkyl (of 1 to 4 carbon atoms), alkoxy (of 1 to 4 carbon atoms), alkanoyloxy, aminocarbonyl, or carboxy groups.

The expression "a 4,5,6 or 7-membered heterocycle" (referred to as "$R_x$") refers to substituted and unsubstituted, aromatic and non-aromatic groups containing one or more (preferably 1, 2 or 3) nitrogen, oxygen or sulfur atoms. Exemplary substituents are oxo (=O), halogen, hydroxy, nitro, amino, cyano, trifluoromethyl, alkyl of 1 to 4 carbons, alkoxy of 1 to 4 carbons, alkylsulfonyl, phenyl, substituted phenyl, 2-furfurylideneamino

( 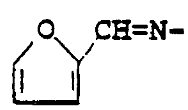 ),

benzylideneamino and substituted alkyl groups (wherein the alkyl group has 1 to 4 carbons). One type of "4,5,6 or 7-membered heterocycle" is the "heteroaryl" group. The term "heteroaryl" refers to those 4,5,6 or 7-membered heterocycles which are aromatic. Exemplary heteroaryl groups are substituted and unsubstituted pyridinyl, furanyl, pyrrolyl, thienyl, 1,2,3-triazolyl, 1,2,4-triazolyl, imidazolyl, thiazolyl, thiadiazolyl, pyrimidinyl, oxazolyl, triazinyl, and tetrazolyl. Exemplary nonaromatic heterocycles (i.e., fully or partially saturated heterocyclic groups) are substituted and unsubstituted azetidinyl, oxetanyl, thietanyl, piperidinyl, piperazinyl, imidazolidinyl, oxazolidinyl, pyrrolidinyl, tetrahydropyrimidinyl, dihydrothiazolyl and hexahydroazepinyl. Exemplary of the substituted 4,5,6 or 7-membered heterocycles are 1-alkyl-3-azetidinyl, 2-oxo-1-imidazolidinyl, 3-alkylsulfonyl-2-oxo-1-imidazolidinyl, 3-benzylideneamino-2-oxo-1-imidazolidinyl, 3-alkyl-2-oxo-1-imidazolidinyl, 3-phenyl (or substituted phenyl)-2-oxo-1-imidazolidinyl, 3-benzyl-2-oxo-1-imidazolidinyl, 3-(2-aminoethyl)-2-oxo-1-imidazolidinyl, 3-amino-2-oxo-1-imidazolidinyl, 3-[(alkoxycarbonyl)-amino]-2-oxo-1-imidazolidinyl, 3-[2-[(alkoxycarbonyl)amino]ethyl]-2-oxo-1-imidazolidinyl, 2-oxo-1-pyrrolidinyl, 2-oxo-3-oxazolidinyl, 4-hydroxy-6-methyl-2-pyrimidinyl, 2-oxo-1-hexahydroazepinyl, 2-oxo-3-pyrrolidinyl, 2-oxo-3-tetrahydrofuranyl, 2,3-dioxo-1-piperazinyl, 2,5-dioxo-1-piperazinyl, 4-alkyl-2,3-dioxo-1-piperazinyl, and 4-phenyl-2,3-dioxo-1-piperazinyl.

The term "substituted amino" refers to a group having the formula $-NX_8X_9$ wherein $X_8$ is hydrogen, alkyl, phenyl, substituted phenyl, phenylalkyl or (substituted phenyl)alkyl, and $X_9$ is alkyl, phenyl, substituted phenyl, phenylalkyl, (substituted phenyl)alkyl, hydroxy, cyano, alkoxy, phenylalkoxy, or amino ($-NH_2$).

The term "acyl" refers to all organic radicals derived from an organic acid (i.e., a carboxylic acid) by removal of the hydroxyl group. Certain acyl groups are, of course, preferred but this preference should not be viewed as a limitation of the scope of this invention. Exemplary acyl groups are those acyl groups which have been used in the past to acylate β-lactam antibiotics including 6-aminopenicillanic acid and derivates and 7-aminocephalosporanic acid and derivatives; see, for example Cephalosporins and Penicillins, edited by Flynn, Academic Press (1972), German Offenlegungsschrift 2,716,677, published October 10, 1978, Belgian patent 867,994, published December 11, 1978, United States patent 4,152,432, issued May 1, 1979, United States patent 4,172,199, issued October 23, 1979, and British patent 1,348,894, published March 27, 1974. The portions of these references describing various acyl groups re incorporated herein by reference. The following list of acyl groups is presented to further exemplify the term "acyl"; it should not be regarded as limiting that term. Exemplary acyl groups are:

(a) Aliphatic groups having the formula

$$R_a-\overset{O}{\overset{\|}{C}}-$$

wherein $R_a$ is alkyl; cycloalkyl;alkoxy; alkenyl; cycloalkenyl; cyclohexadienyl; or alkyl or alkenyl substituted with one or more halogen, cyano, nitro, amino, mercapto, alkylthio, or cyanomethyltheio groups.

(b) Carbocyclic aromatic groups having the formula

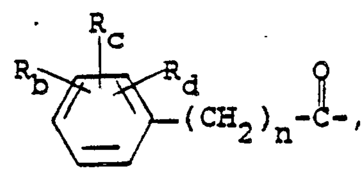

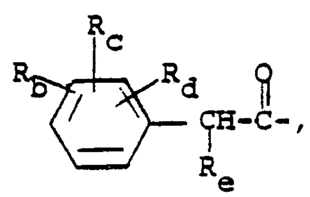

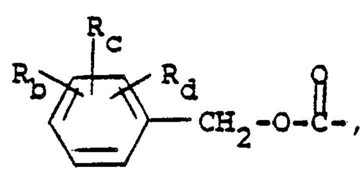

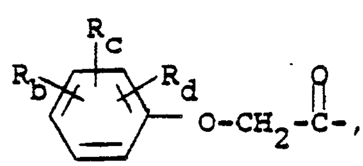

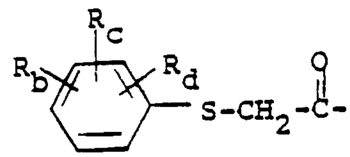

or

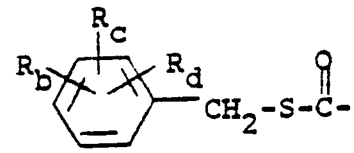

wherein n is 0, 1, 2 or 3; $R_b$, $R_c$, and $R_d$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_e$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl groups include those having the formula

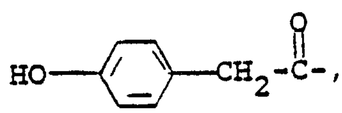

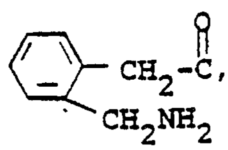

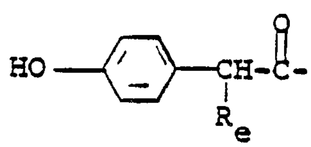

($R_e$ is preferably a carboxyl salt or sulfo salt) and

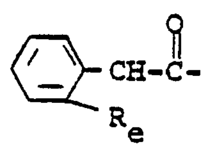

($R_e$ is preferably a carboxyl salt or sulfo salt).

(c) Heteroaromatic groups having the formula

$R_f-(CH_2)_n-\overset{\overset{O}{\|}}{C}-$,

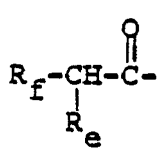

,

$R_f-O-CH_2-\overset{\overset{O}{\|}}{C}-$ ,

$R_f\text{-}S\text{-}CH_2\text{-}\overset{O}{\overset{||}{C}}\text{-}$ ,

$R_f\text{-}\overset{O}{\overset{||}{C}}\text{--}\overset{O}{\overset{||}{C}}\text{-}$ ,

wherein n is 0, 1, 2 or 3; $R_e$ is as defined above; and $R_f$ is a substituted or unsubstituted 5-, 6- or 7-membered heterocyclic ring containing 1, 2, 3 or 4 (preferably 1 or 2) nitrogen, oxygen and sulfur atoms. Exemplary heterocyclic rings are thienyl, furyl, pyrrolyl, pyridinyl, pyrazolyl, pyrazinyl, thiazolyl, pyrimidinyl, thiadiazolyl and tetrazolyl. Exemplary substituents are halogen, hydroxyl, nitro, amino, protected amino, cyano, trifluoromethyl, alkyl of 1 to 4 carbon atoms, alkoxy of 1 to 4 carbon atoms, or

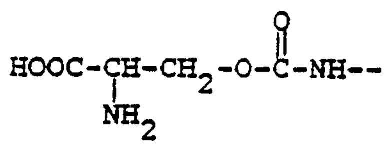 .

Preferred heteroaromatic acyl groups include those groups of the above formulas wherein $R_f$ is 2-amino-4-thiazolyl,
2-amino-5-halo-4-thiazolyl, 4-aminopyrimidin-2-yl, 5-amino-1,2,4-thiadiazol-3-yl, 2-thienyl, 2-furanyl, or 6-aminopyridin-2-yl.

(d) [[(4-Substituted-2,3-dioxo-1-piperazinyl)carbonyl]amino]arylacetyl groups having the formula

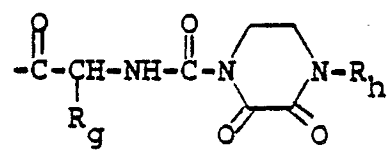

wherein $R_g$ is an aromatic group (including carbocyclic aromatics such as those of the formula

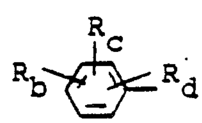

and heteroaromatics as included within the definition of $R_f$); and $R_h$ is alkyl, substituted alkyl (wherein the alkyl group is substituted with one or more halogen, cyano, nitro, amino or mercapto groups), arylmethyleneamino (i.e., $-NH=CH-R_g$ wherein $R_g$ is as defined above), arylcarbonylamino (i.e., $-NH\text{-}\overset{O}{\overset{||}{C}}\text{-}R_g$ wherein $R_g$ is as defined above) or alkylcarbonylamino.

Preferred [[(4-substituted-2,3-dioxo-1-piperazinyl)carbonyl]amino]arylacetyl groups include those wherein $R_h$ is ethyl, phenylmethyleneamino or 2-furylmethyleneamino.

(e) (Substituted oximino)arylacetyl groups having the formula

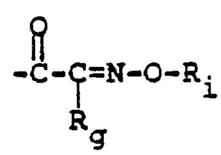

wherein $R_g$ is as defined above and $R_i$ is hydrogen, alkyl, cycloalkyl,

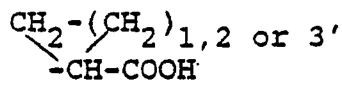

2-pyrazolylmethyl, (2-oxo-3-pyrrolidinyl)methyl, alkylaminocarbonyl, arylaminocarbonyl (i.e., $-\overset{O}{C}-NH-R_g$ is as defined above) or substituted with one or more halogen, cyano, nitro, amino, mercapto, alkylthio, aromatic group (as defined by $R_g$), carboxyl (including salts thereof), amido, alkoxycarbonyl, phenylmethoxycarbonyl, diphenylmethoxycarbonyl, phenylmethoxycarbonyl, dihydroxyphosphinyl, hydroxy-(phenylmethoxy)phosphinyl, dialkoxyphosphinyl or tetrazolyl substituents).

Preferred (substituted oxyimino) arylacetyl groups include those wherein $R_g$ is 2-amino-4-thiazolyl. Also preferred are those groups wherein $R_i$ is methyl, ethyl, carboxymethyl, 1-carboxy-1-ethyl (R and S isomer) 1-carboxy-1-methylethyl, 2,2,2-trifluoroethyl or 1-carboxycyclopropyl, 1-carboxycyclobutyl or 1-carboxycyclopentyl.

(f) (Acylamino)arylacetyl groups having the formula

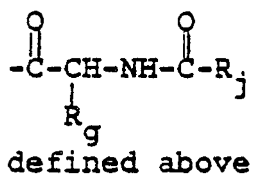

wherein $R_g$ is as defined above and $R_j$ is

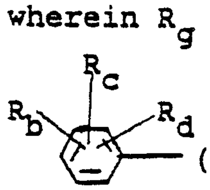 $(CH_2)_n$-O-, amino, alkylamino,

(cyanoalkyl)-amino, amido, alkylamido, (cyanoalkyl)amido,

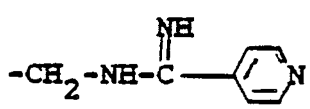

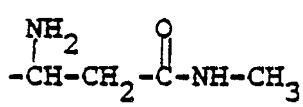,

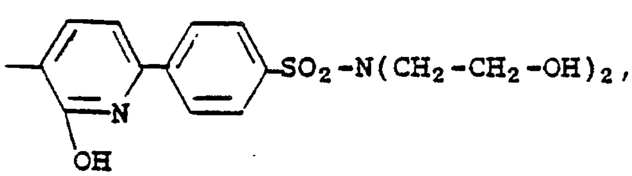,

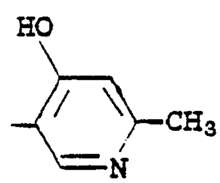,

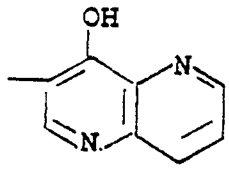 , 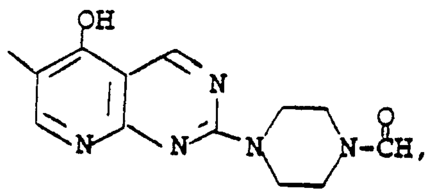 , or

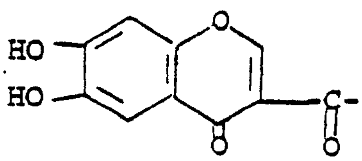 .

Preferred (acylamino)arylacetyl groups of the above formula include those groups wherein $R_j$ is amino or amido. Also preferred are those groups wherein $R_g$ is phenyl or 2-thienyl.

(g) [[[3-Substituted-2-oxo-1-imidazolidinyl]carbonyl]amino]arylacetyl groups having the formula

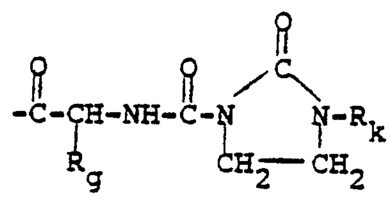

wherein $R_g$ is as defined above and $R_k$ is hydrogen, alkylsulfonyl, arylmethyleneamino (i.e., $-N=CH-R_g$ wherein $R_g$ is as defined above),

$-\overset{O}{\overset{\|}{C}}-R_m$ (wherein $R_m$ is hydrogen, alkyl or halogen substituted alkyl), aromatic group (as defined by $R_g$ above), alkyl or substituted alkyl (wherein the alkyl group is substituted with one or more halogen, cyano, nitro, amino or mercapto groups).

Preferred [[[3-substituted-2-oxo-1-imidazolidinyl]carbonyl]amino]arylacetyl groups of the above formula include those wherein $R_g$ is phenyl or 2-theinyl. Also preferred are those groups wherein $R_k$ is hydrogen, methylsulfonyl, phenylmethyleneamino or 2-furylmethyleneamino.

The compounds of this invention form basic salts with various inorganic and organic bases which are also within the scope of this invention. Such salts include ammonium salts, alkali metal salts, alkaline earth metal salts, salts with organic bases, e g., dicyclohexylamine, benzathine, N-methyl-D-glucamine, hydrabamine and the like. The pharmaceutically acceptable salts are preferred, although other salts are also useful, e.g., in isolating or purifying the product.

Some of the compounds of this invention may be crystallized or recrystallized from solvents containing water. In these cases, water of hydration may be formed. This invention contemplates stoichiometric hydrates as well as compounds containing variable amounts of water that may be produced by processes such as lyophilization.

The β-lactams of formula I contain at least one chiral center--the carbon atom in the 3-position of the β-lactam nucleus to which the acylamino substituent ("$R_1$-NH-") is attached. This invention is directed to those β-lactams which have been described above, wherein the stereochemistry at the chiral center in the 3-position of the β-lactam nucleus is the same as the configuration at the carbon atom in the 6-position of naturally occurring penicillins (e.g., penicillin G) and as the configuration at the carbon atom in the 7-position of naturally occurring cephamycins (e.g., cephamycin C). Also included within the scope of this invention are racemic mixtures which contain the above-described β-lactams.

It is understood that all hydroxypyridone compounds of this invention may exist as their dihydroxypyridine tautomers.

The β-lactams of formula I, and pharmaceutically acceptable salts thereof, have activity against gram-positive and gram-negative organisms. The compounds of this invention can be used as agents to combat

bacterial infections (including urinary tract infections and respiratory infections) in mammalian species, such as domesticated animals (e.g., dogs, cats, cows, horses, and the like) and humans.

For combating bacterial infections in mammals, a compound of this invention can be administered to a mammal in need thereof in an amount of about 1.4 mg/kg/day to about 350 mg/kg/day, preferably about 14 mg/kg/day to about 100 mg/kg/day. All modes of administration which have been used in the past to deliver penicillins and cephalosporins to the site of the infection are also contemplated for use with β-lactams of this invention. Such methods of administration include oral, intravenous, intramuscular, and as a suppository.

The β-lactams of formula I can be prepared from a 3-protected amino-2-azetidinone having the formula

II

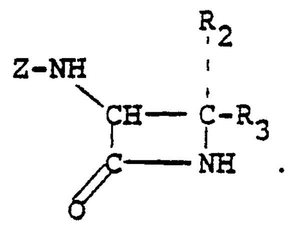

In formula II, and throughout the specification, the symbol "Z" refers to an amino protecting group. These groups are well known in the field of β-lactam chemistry, and the particular group chosen is not critical. Benzyloxycarbonyl, trityl, and t-butoxycarbonyl are exemplary protecting groups. The reaction of a β-lactam of formula II with an isocyanate having the formula

III $O=C=N-SO_2-L$ ,

wherein L is a leaving group such as chlorine, yields the corresponding compound having the formula

IV

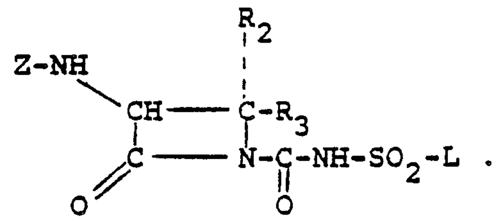

The reaction is preferably run in an inert organic solvent, e.g., ethyl acetate, tetrahydrofuran, dimethoxyethane, dichloromethane, acetonitrile or mixtures of these solvents. Displacement of the leaving group "L" with the desired group "R" can be accomplished using the appropriate nucleophile having the formula

V $R_h$,

optionally in the presence of a base (e.g., triethylamine), and yields the corresponding compound having the formula

VI

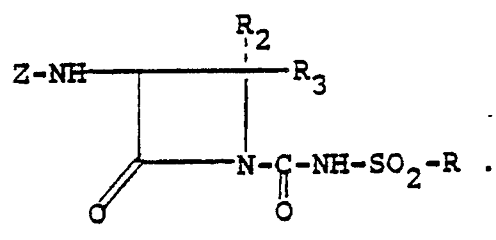

Alternatively, the displacement of the leaving group can be accomplished by reaction of a compound of formula IV with a protected form of a compound of formula V. Following the displacement reaction, the protecting groups can be removed using art-recognized techniques to yield a compound of formula VI.

Protected forms of a compound of formula V, and of all reactants described herein which contain a 3-hydroxy-4-pyridone moiety, include those compounds wherein the hydroxyl group is protected, those compounds wherein the hydroxyl group and the ring nitrogen are protected, and those compounds wherein both pyridone oxygens are protected. Exemplary protecting groups are silyl (e.g., trimethylsilyl), benzyl and acyl (e.g., acetyl). If silyl is used, later deprotection can be accomplished using hydrolysis or fluoride

mediated cleavage. If benzyl is used, later deprotection can be accomplished by hydrogenolysis. If acyl is used, later deprotection can be accomplished by hydrolysis.

Deprotection of a compound of formula VI using conventional techniques yields the corresponding key intermediate having the formula

VII

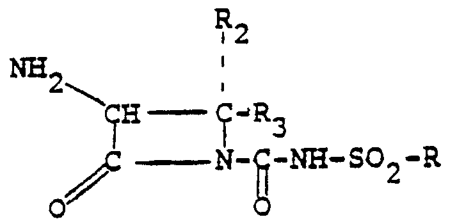,

or a salt thereof. The particular deprotection reaction used will, of course, depend on the protecting group ("Z") present. If, for example, Z is a t-butoxycarbonyl protecting group, deprotection can be accomplished by treatment of a compound of formula VI with acid (e.g., formic acid or trifluoroacetic acid). If, for example, Z is a benzyloxycarbonyl protecting group, deprotection can be accomplished by catalytic hydrogenation of a compound of formula VI. Alternatively, the Z protecting group can be removed simultaneously with the other pyridone protecting groups immediately following the above-described displacement reaction.

Well known acylation techniques can be used to convert an intermediate of formula VII to a corresponding product of formula I. Exemplary techniques include reaction of a compound of formula VII with a carboxylic acid ($R_1$-OH), or corresponding carboxylic acid halide or carboxylic acid anhydride. The reaction with a carboxylic acid proceeds most readily in the presence of a carbodiimide such as dicyclohexylcarbodiimide and a substance capable of forming an active ester in situ such as N-hydroxybenzotriazole. In those instances where the acyl group ($R_1$) contains reactive functionality (such as amino or carboxyl groups) it may be necessary to first protect those functional groups, then carry out the acylation reaction, and finally deprotect the resulting product.

An alternative procedure for preparing the compounds of formula I comprises first acylating (acylation techniques have been described above) a 3-amino-2-azetidinone having the formula

VIII

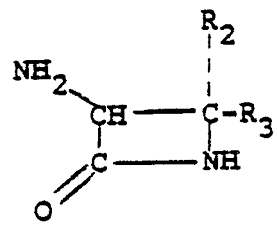

to yield an intermediate having the formula

IX

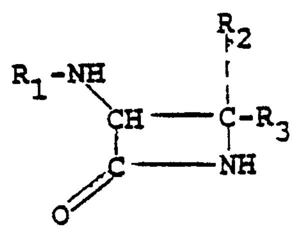.

A - $\overset{\overset{O}{\|}}{C}$-NH-$SO_2$-R activating group can be introduced in the 1-position of a compound of formula IX (using the procedures described above) to obtain the corresponding product of formula I. In those instances wherein the acyl side-chain "$R_1$" contains reactive functionality (such as amino groups), it may be necessary to first protect those functional groups, then carry out the addition of the activating group in the 1-position, and finally deprotect the resulting product.

Still another synthesis for the preparation of compounds of formula I comprises the use of a 3-azido-2-

azetidinone having the formula

X

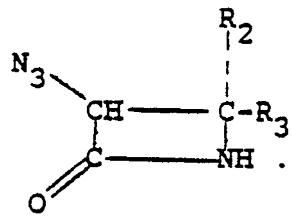

O
A - C -NH-$SO_2$-R activating group can be introduced in the 1-position of a compound of formula X (using the procedures described above) to obtain the corresponding compound having the formula

XI

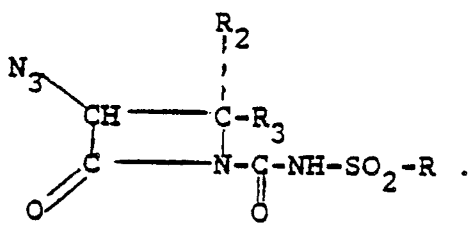

Reduction of an intermediate of formula XI yields the corresponding intermediate having the formula

VII

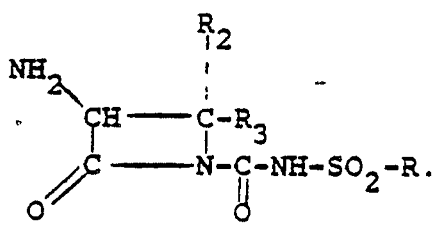

The reduction can be accomplished by catalytic (e.g., palladium on charcoal or platinum oxide) hydrogenation or with reducing agents such as zinc or triphenylphosphine. As described above, from these key intermediates (compounds of formula VII), using conventional acylation techniques, it is possible to prepare the products of formula I.

Alternatively, a 3-azido-2-azetidinone of formula X can be reduced to the corresponding 3-amino-2-azetidinone having the formula

VIII

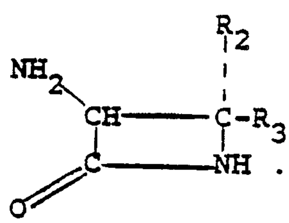

The reduction can be accomplished by catalytic (e.g., palladium on charcoal or platinum oxide) hydrogenation or with reducing agents such as zinc or triphenylphosphine. A 3-amino-2-azetidinone of formula VIII can be reacted as described above (i.e., first acylated and then treated as described above to introduce a
O
- C-NH-$SO_2$-R activating group in the 1-position) to yield the products of formula I.

Still another synthesis for preparing the compounds of formula I wherein $R_2$ and $R_3$ are each hydrogen utilizes a 6-acylaminopenicillanic acid having the formula

XII

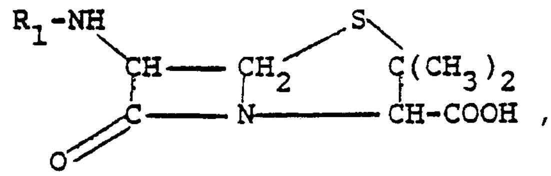

or a salt thereof, as the starting material. By adapting procedures described in the literature, 3-acylamino-2-azetidinone can be obtained from the corresponding 6-acylaminopenicillanic acid of formula XII: see, for example, Chem. Soc. Special Publication No. 28, pg. 288 (1977), The Chemistry of Penicillins, Princeton University Press, pg. 257, and Synthesis, 494 (1977).

As described in the literature 6-acylaminopenicillanic acid, or a salt thereof, can be desulfurized to yield a compound having the formula

XIII

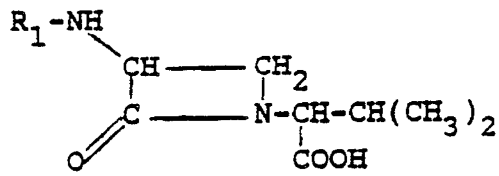

by reduction using Raney nickel. The reaction can be run in water under reflux conditions.

Replacement of the carboxyl group of a compound of formula XIII with an acetate group followed by hydrolysis yields the corresponding 3-acylamino-2-azetidinone having the formula

XIV

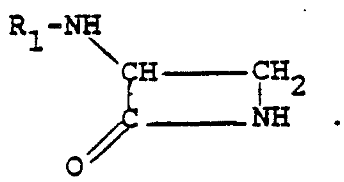

Treatment of a compound of formula XIII with cupric acetate and lead tetraacetate in an organic solvent (e.g., acetonitrile) replaces the carboxyl group with an acetate group. Hydrolysis of the resulting compound can be accomplished using potassium carbonate in the presence of sodium borohydride.

A - $\overset{O}{\overset{\|}{C}}$ -NH-$SO_2$-R activating group can be introduced in the 1-position of a compound of formula XIV (yielding products of formula I wherein $R_2$ and $R_3$ are each hydrogen) using the procedures described above.

Still another variation of the above-described synthetic routes for preparing a compound of formula I wherein $R_2$ and $R_3$ are each hydrogen comprises first desulfurizing 6-aminopenicillanic acid, acylating the resulting compound to yield a compound of formula XIII and then proceeding as described above to obtain first a 3-acylamino-2-azetidinone of formula XIV and then a product of formula I.

The azetidinones of formula I can also be prepared from amino acids having the formula

XV

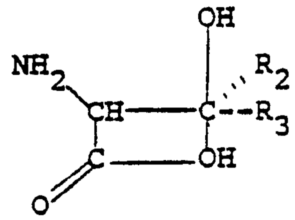

The amino group is first protected (with a protecting group "Z", e.g., t-butoxycarbonyl). The carboxyl group of the protected amino acid is then reacted with an amine having the formula

XVI $Z^1$-O-$NH_2$,

wherein $Z^1$ is alkyl, benzyl or triphenylmethyl, in the presence of a carbodiimide to yield a compound having the formula

XVII

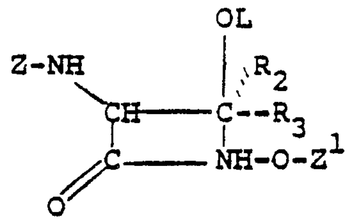

The hydroxyl group of a compound of formula XVII is converted to a leaving group ("OL) with a reagent, such as methanesulfonyl chloride or pyridine-$SO_3$ complex.

The fully protected compound having the formula

XVIII

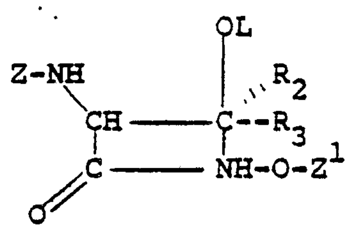

is cyclized by treatment with base, e.g., potassium carbonate. The reaction is preferably carried out in an organic solvent or an organic solvent/water mixture under reflux conditions, and yields a compound having the formula

XIX

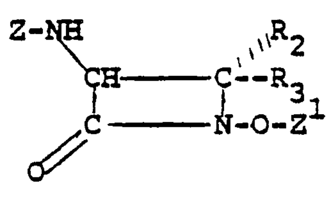

Alternatively, cyclization of a compound of formula XVII can be accomplished without first converting the hydroxyl group to a leaving group. Treatment of a compound of formula XVII with triphenylphosphine and diethylazodicarboxylate, yields a compound of formula XIX.

Exemplary procedures for the conversion of a compound of formula XVIII to a compound of formula XIX are described in J. Amer. Chem. Soc., 102, 7026 (1980) and J. Org. Chem., 47, 5160 (1982).

Both of the methods disclosed above for ring closure of a compound of formula XVII result in the inversion of the stereochemistry at the carbon atom bearing the $R_2$ and $R_3$ substituents when $R_2$ and $R_3$ are not the same.

Removal of the protecting group from the 1-position of an azetidinone of formula XIX can be

accomplished via sodium reduction when $Z^1$ is alkyl, and yields an intermediate having the formula

II

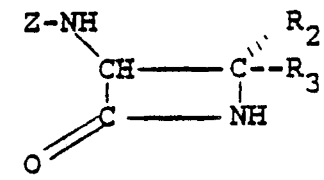

(at least one of $R_2$ and $R_3$ is hydrogen). If $Z^1$ is benzyl, catalytic (e.g., palladium on charcoal) hydrogenation will initially yield the corresponding N-hydroxy compound, which upon treatment with titanium trichloride yields an intermediate of formula II. If $Z^1$ is triphenylmethyl, formic acid or 70% acetic acid/water will initially yield the corresponding N-hydroxy compound.

A - $\overset{O}{\overset{\|}{C}}$ -NH-$SO_2$-R activating group can be introduced in the 1-position of a compound of formula II using the procedures described above, and the resulting compound can be deprotected and acylated.

The nucleophiles RH of formula V can be prepared in a similar manner as described in U.S. Serial No. 907,441 filed September 15, 1986. The nucleophiles in the present application contain a "Y" group, hereinbefore defined.

Alternatively, the compounds of formula I can be formed by reacting a compound having the formula

XX

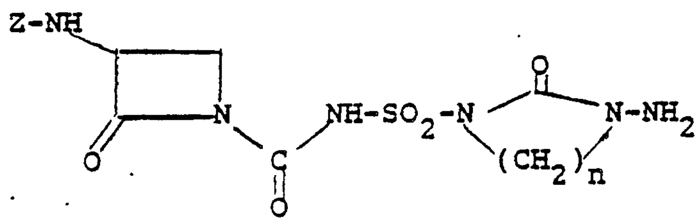

with a compound of the formula

XXI

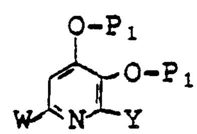

wherein $P_1$ is hydrogen or a hydroxyl protecting group such as benzyl, alkyloxycarbonyl, alkanoyl, phenyl carbonyl or substituted phenyl carbonyl and W is COOH, -CH=CH-COOH or $-\overset{O}{\overset{\|}{C}}-H$

to yield intermediates having the formula

XXII

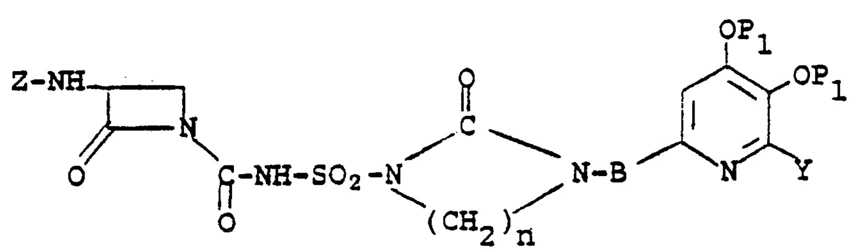

wherein B is

H O H O
I II I II
- N - C -, - N - C -CH=CH-, or -N=CH-. The Y moiety can be optionally protected. Intermediate compound XXII can be fully deprotected in one step by conventional methods to yield intermediates of the formula

XXIII

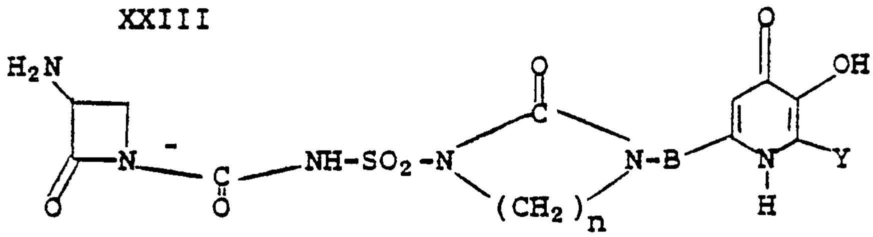

or a salt thereof. Acylation with a compound of the formula

XXIV $R_1$-OH

or activated derivatives thereof will yield compounds of formula I.

It is also possible to synthesize the compounds of formula I by reacting compounds of the formula

XXV

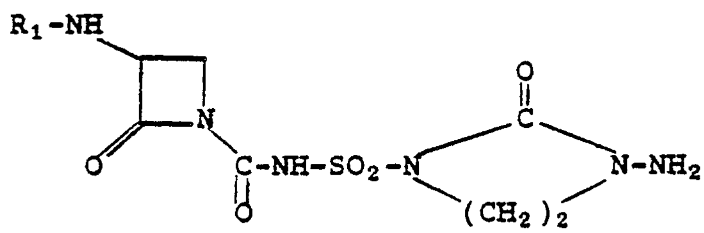

with compounds of the formula

XXVI

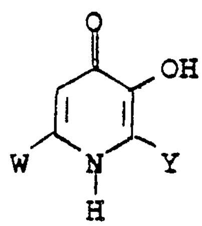

wherein W is -COOH, -CH=CH-COOH or
O
II
- C -H by conventional coupling methods. The Y moiety can be optionally protected. The above described methods involving the compounds of formula XX or XXV are also applicable by substituting the compounds represented by the formula

XXVII

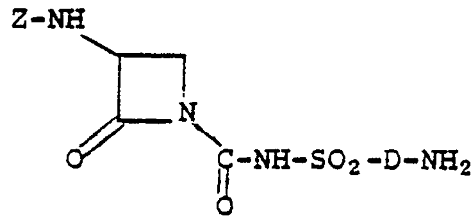

wherein D is NH, 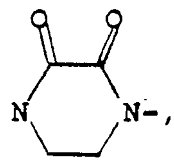, 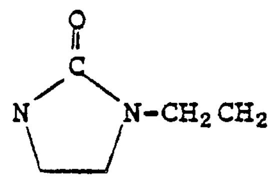, or

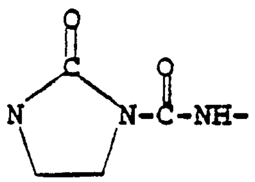.

Compounds of formula XX and XXV can be prepared by reacting a compound of formula IV or a compound of formula IV when Z equals $R_1$ in a solvent, such as methylene chloride, tetrahydrofuran, acetonitrile or ethyl acetate, with compounds of the formula

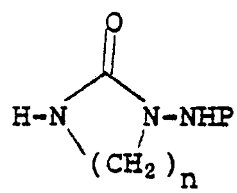.

and subsequent deprotection of P. P is an amino protecting group such as tert-butyloxycarbonyl or benzyloxycarbonyl.

Compounds of formula I wherein Y is $CH_2X$ and X is

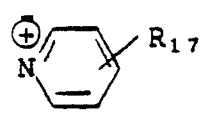

can be prepared by reacting a compound of the formula

XXVIII

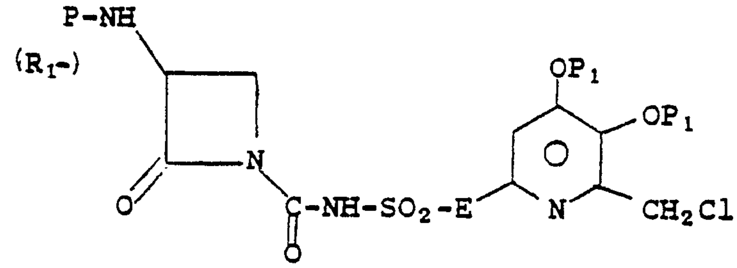

wherein E is 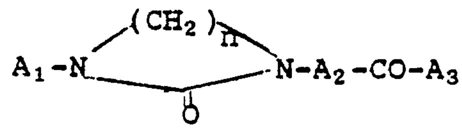

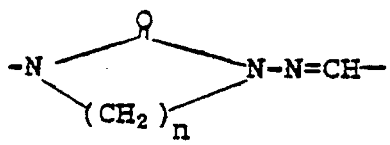 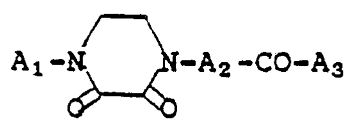

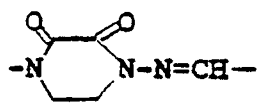 $-NH-A_4$

with pyridine or substituted pyridines.

Compounds of formula I wherein Y is $CH_2NH-\underset{\underset{O}{\|}}{C}-NH$-lower alkyl or $CH_2NH-\underset{\underset{S}{\|}}{C}-NH$-lower alkyl can be prepared from the corresponding compound of formula I wherein Y is $CH_2NH_2$ by procedures known to one skilled in the art.

Compounds of formula I wherein Y is $CONH_2$ can be prepared from the corresponding compound of formula I wherein Y is COOH by procedures known to one skilled in the art.

Compounds of formula I wherein Y is $\underset{\underset{S}{\|}}{C}-NH_2$ can be prepared from the corresponding compound of formula I wherein Y is C≡N by procedures known to one skilled in the art.

This invention includes the novel hydroxypyridones or the deprotected hydroxypyridones represented by the formulas

XXIX

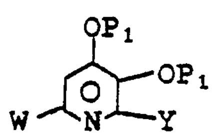

XXIXa

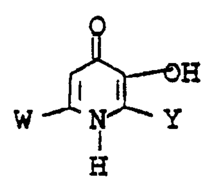

wherein W is COOH, -HC=CH-COOH or

$\overset{O}{\overset{\|}{C}}$-H. Compounds of XXIX may be converted to compounds of formula XXIXa when $P_1$ is a protecting group using standard methods known in the art. These novel hydroxypyridones are used to synthesize the nucleophile(V) RH. Compounds XXIX and XXIXa are prepared by reacting maltol with sodium methoxide and benzyl bromide to obtain the compound having the formula

XXX

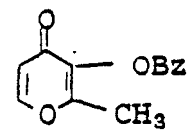

wherein Bz is benzyl. Compound XXX is reacted with ammonia in ethyl alcohol to yield

XXXI

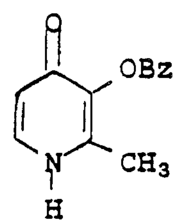

Compound XXXI is reacted with benzylhalides in the presence of a base such as potassium carbonate or cesium carbonate in a solvent such as methanol, ethanol, or isopropanol at room temperature up to about 80° C to yield the compound of the formula

XXXII

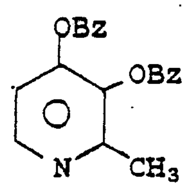

Better yields of compound XXXII are obtained by reacting compound XXXI with benzyl alcohol, triphenylphosphine and diethyl azodicarboxylate in solvents such as tetrahydrofuran (THF) or dioxane. Compound XXXII is reacted with a peracid such as meta-chloroperbenzoic acid (MCPBA) in solvents such as $CHCl_3$ or dichloromethane to yield the compound of the formula

XXXIII

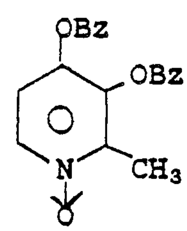

Compound XXXIII is heated with acetic anhydride to yield the compound having the formula

XXXIV

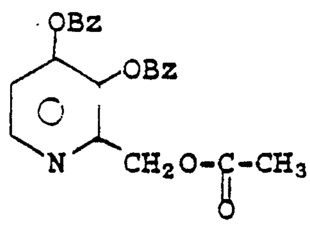

Compound XXXIV is reacted with MCPBA in chloroform or $CH_2Cl_2$ at room temperature to about 80° C to yield the compound having the formula

XXXV

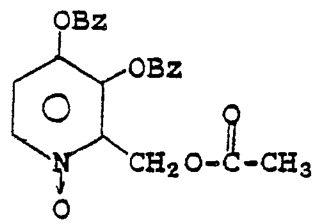

Compound XXXV is reacted with dimethylsulfate in acetonitrile at reflux temperature and the intermediate methoxy-2-[(acetyloxy)methyl]-3,4-bis(phenylmethoxy) pyridinium monomethylsulfate is isolated and then reacted with potassium cyanide in THF/$C_2H_5OH$/$H_2O$ at -10° C to -5° C to yield the compound having the formula

XXXVI

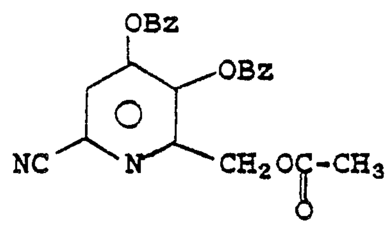

An alternate synthesis of compound XXXVI is to react compound XXXV with dimethylcarbamide chloride in acetonitrile and isolating the intermediate 1-(dimethylaminocarbonyloxy)-2-[(acetyloxy)methyl]-,3,4-bis (phenylmethoxy) pyridinium chloride and reacting this intermediate with trimethylsilylcyanide and triethylamine in acetonitrile at an elevated temperature to yield compounds of formula XXXVI. Another alternate synthesis is reacting a compound of the formula XXXV with trimethylsilylcyanide and triethylamine in a solvent like acetonitrile or $CH_2Cl_2$ at reflux temperature. Saponification of the compounds of formula XXXVI with a large excess of lithium hydroxide at the reflux temperature in solvent mixtures such as $H_2O$/THF or water/dioxane forms compound XXXVII after acidification

XXXVII

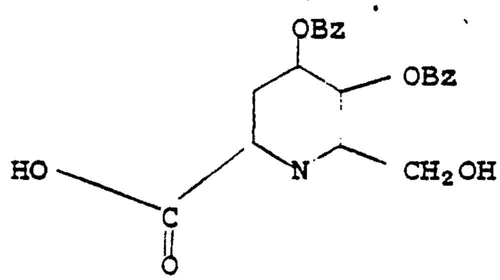

Compound XXXIII can be converted to compound XXXVIII

XXXVIII

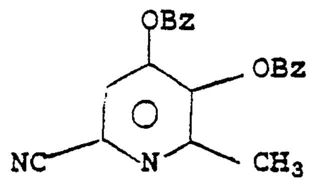

under similar conditions used to transform compound XXXV to compound XXXVI. Saponifications of compound XXXVIII with bases such as KOH,NaOH or preferred LiOH in mixtures of THF/water or dioxane/water and acidification yields a compound having the formula

XXXIX

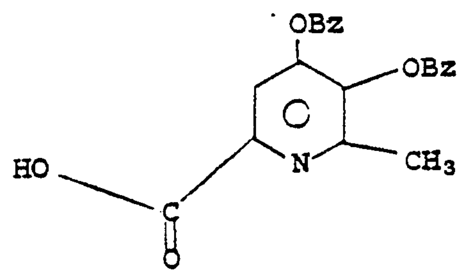

Compound XXXVIII can be reacted with methanol/HCl gas at room temperature to yield the methyl ester having the formula

XXXX

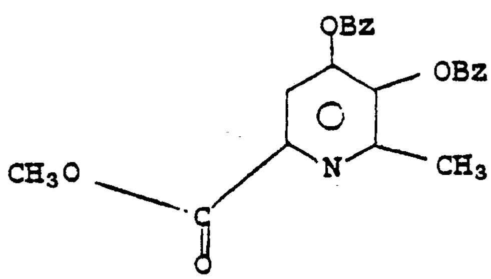

Compound XXXX can also be obtained from compound XXXIX when it is reacted with methanol/HCl. Compound XXXX is reacted with MCPBA in chloroform at room temperature to yield the compound having the formula

XXXXI

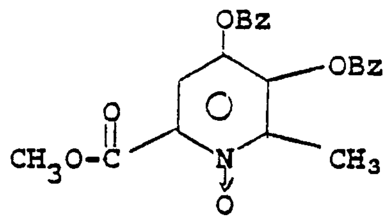

Heating of compound XXXXI with excess acetic anhydride yields a compound having the formula

XXXXII

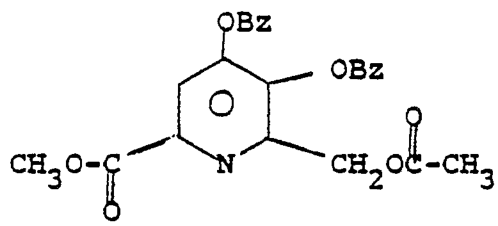

Saponification of compound XXXXII with bases such as KOH, NaOH or LiOH in water/THF or water/dioxane at the reflux temperature results in the salt of compound XXXVII which is then acidified to yield compound XXXVII.

Compound XXXVII can be converted to the novel intermediates of formula XXIX by first protecting the carboxylic group and then reacting with known functional groups to result in compounds of XXIX wherein Y is $CH_2OR_{12}$. For example, the protected form of compound XXXVII can be reacted with isocyanates to yield compounds of XXIXa wherein Y is $CH_2O\underset{O}{\underset{\|}{C}}$ -NH-lower alkyl.

Alkylation of the protected form of XXXVII with alkyl halogenides and bases yields compounds of XXIX wherein Y is $CH_2O$-lower alkyl and P is benzyl.

Compound XXXVII can be reacted with benzyl bromide or benzyl chloride in dimethylformamide and one equivalent of base such as potassium carbonate or triethylamine at room temperature to form compounds having the formula

XXXXIII

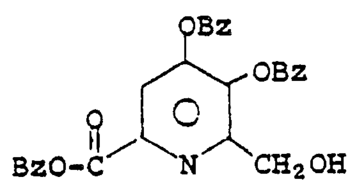

Compound XXXVII can be reacted with thionylchloride to give a compound having the formula

XXXXIV

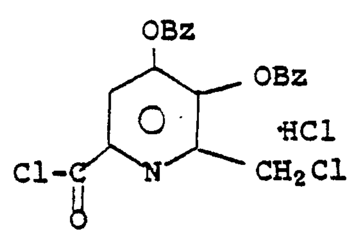

Compound XXXXIV can be allowed to stand in anhydrous methanol for 24 hours to give a compound of the formula

XXXXV

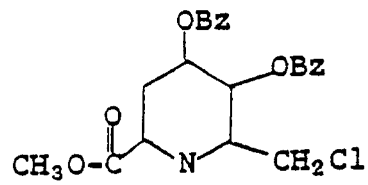

Compound XXXXV can be reacted with a source of azide ion ($N_3$) in DMF in the presence of catalytic amounts of potassium iodide to form a compound having the formula

XXXXVI

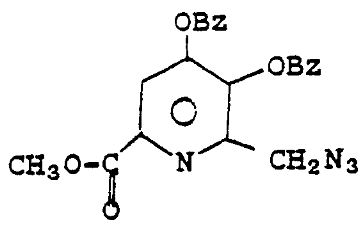

Saponification of compound XXXXVI with potassium hydroxide in mixtures of water with organic solvents such as ethanol, THF, dioxane, methanol at room temperature, followed by acidification gives a compound having the formula

XXXXVII

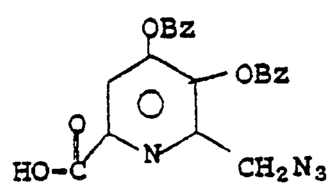

Using conventional methods for reducing azides such as triethylamine/$H_2S$ gives the compound having the formula

XXXXVIII

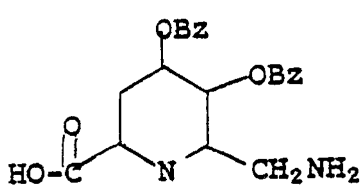

Compound XXXXVIII or the carboxylic group protected derivative of XXXXVIII can be converted to the novel intermediates of XXIX wherein Y is $CH_2$-NH-$R_{13}$ wherein P is benzyl.

For example, compound XXXXVIII can be heated with potassium isocyanate or isothiocyanate, in water/dioxane to yield the novel intermediate XXIX wherein Y is $CH_2NH-\underset{O}{\underset{\|}{C}}-NH_2-$

or $CH_2NH-\underset{S}{\underset{\|}{C}}-NH_2-$

or a protected form of compound XXXXVIII (COOP) can be reacted with isocyanates or isothiocyanates to novel intermediates XXIXa wherein Y is $CH_2NH-\underset{\underset{S}{\|}}{C}-NH$ lower alkyl

or $CH_2NH \overset{C}{\underset{S}{\|}}$ NH-lower alkyl.

Compound XXXXV can be reacted with nucleophiles in solvents such as DMF and dimethylacetamide to form compounds of formula XXIX wherein Y is C≡N, $(S)_mR_{14}$, $(O)_mR_{14}$ and S-C≡N. Further transformation of a compound of formula XXIX wherein Y is C≡N will yield compounds of formula XXIXa wherein Y is $CH_2COOR_{15}$ and $CONHR_{16}$.

Compound XXXXIII can be oxidized for example with activated manganese oxide in $CH_2Cl_2$ to yield a compound of the formula

XXXXIX

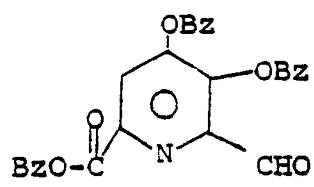

Transformation of compound XXXXIX by conventional procedures produces compounds of formula XXIXa wherein Y is CH = NO-loweralkyl, C≡N, $CONH_2$, $CHF_2$, CH = $CHR_9$, $CONHOR_{10}$ and $CSNHR_{11}$.

Specifically, compound XXXXIX can be reacted with hydroxylamine or a protected hydroxylamine to give an intermediate with Y being CH = NOH. This intermediate can be dehydrated to a compound of formula XXIX with Y being C≡N. Compound XXXXIX can be reacted with diethylamino sulfuryltrifluoride in $CH_2Cl_2$ to give a compound of formula XXIX where Y is $CHF_2$. Reaction of compound XXXXIX with Wittig reagents gives compounds of XXIX wherein Y is CH = $CHR_9$.

Compound XXXXIX can be oxidized with tetrabutylammonium permaganate in pyridine to give a compound of the formula

L

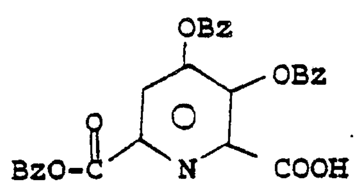

Compound L can be reacted with dicyclohexylcarbodiimide and 4-pyrollidino-pyridine and t-butanol to give a compound of the formula

LI

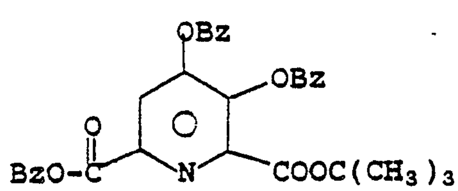

Saponification of LI with KOH in THF and water gives a compound having the formula

LII

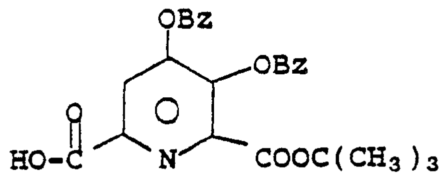

Compound XXXXV can be reacted with pyridines in DMF in the presence of catalytic amounts of potassium iodide to form compounds of XXIX wherein Y is

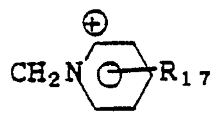

as a salt or a zwitterion.

Reduction of compound XXXX with lithium aluminumhydride in solvents such as ether or THF will produce compounds of the formula

LIII

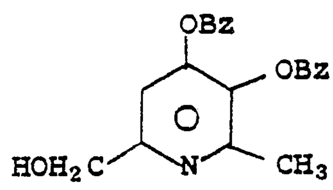

Oxidation of compound LIII by conventional methods such as activated manganese dioxide in solvents such as methylene chloride or acetone gives the aldehyde of the formula

LIV

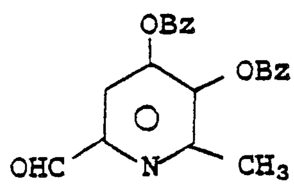

Wittig-Horner reactions with compound LIV will yield compounds of the formula.

LV

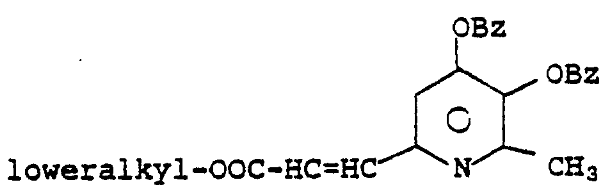

Compound LV can be selectively hydrogenated, for example with a diimine, to form a compound having the formula

LVI

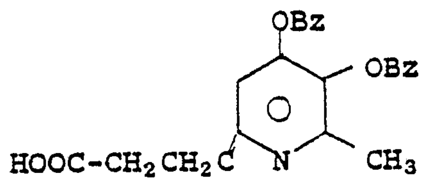

Protection of the aldehyde group in compound LIV with an acetal or cyclic acetal group followed by treatment with a peracid such as MCPBA and deprotection of the acetal group will yield a compound of the formula

LVII

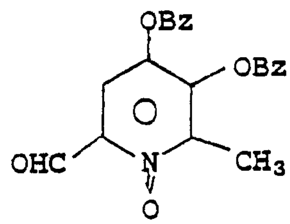

The methyl group in compound LVII can be modified by the same reactions described above wherein compound XXXIII is transformed into compound XXXVII and compound XXXVII is transformed into compound LII to yield compounds of the formula

LVIII

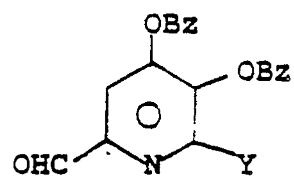

An alternate procedure to the novel intermediates of XXIX and XXIXa is to react O-benzylcomenamic acid with excess benzylbromide in DMF at an elevated temperature to give the fully protected pyridine having the formula

LIX

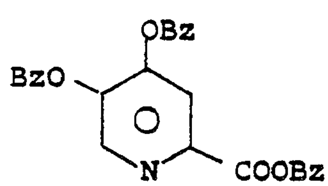

Oxidation of compound LIX with MCPBA in chloroform yields the compound having the formula

LX

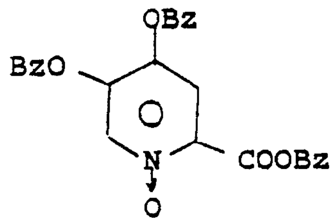

Compound LX can be first reacted with dimethylsulfate in acetonitrile at reflux temperature, isolating the O-methylpyridinium intermediate and reacting this intermediate with KCN in THF/ethanol to form a compound of the formula

LXI

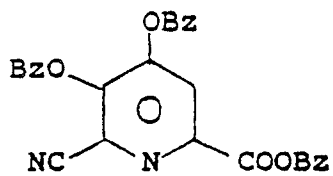

Alternatively, compound LXI can be prepared by reacting compound LX with dimethylaminocarbonylchloride, followed by trimethylsilylcyanide or by reacting compound LX with trimethylsilylcyanide and triethylamine under reflux.

Compound LXI can be converted to novel intermediate XXIXa wherein Y is CN, COOH, $COOR_4$,

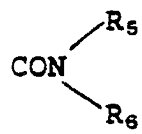 ,

$C\text{-}NH\text{-}R_{11}$
S

and $CONHOR_{10}$ by the methods described above involving compound XXXXIX.

Compound LX can be heated with acetic anhydride to form a compound having the formula

LXII

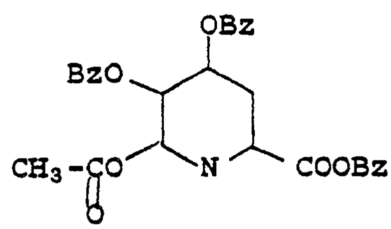

Selective saponification of compound LXII with excess ammonium acetate in dioxane/water gives a compound having the formula

LXIII

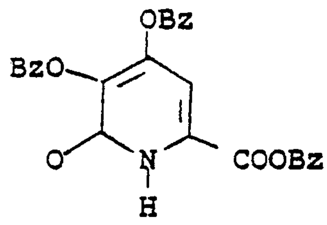

Alkylation of compound LXIII with diazoalkanes in ethanol or with alkylhalides or alkylsulfates and bases such as cesium carbonate yields compounds after saponification having the formula

LXIV

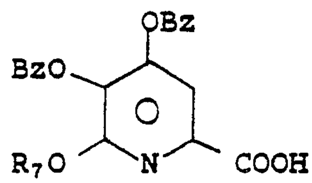

The following examples are specific embodiments of this invention.

Example 1

2-Methyl-3-(phenylmethoxy)-4H-pyran-4-one

11.5g of sodium were dissolved in 300ml $CH_3OH$ and 63g of maltol were added and stirred at room temperature for one hour. 200ml $CH_3OH$ were added and then 102g benzylbromide were dropped in (30 minutes). Refluxing for 3 hours and distilling off the $CH_3OH$ then yielded a residue which was dissolved in 300ml $H_2O$/300ml ethyl acetate. The organic phase was washed with water and evaporated. The remaining oil was distilled in vacuo. B.P. (43mm) = 148-150° C, 81.5g colorless oil of the title compound was isolated.

Example 2

2-Methyl-3-(phenylmethoxy)-4(1H)-pyridone

62g of the product of Example 1 were dissolved in 300ml $C_2H_5OH$ containing 18g $NH_3$. The solution was kept at 110° C for 16 hours in a glass autoclave. After cooling, the $C_2H_5OH$ was distilled off and the crystalline residue filtered off and washed with 50ml $C_2H_5OH$ at 0° C; 49.7g beige crystals were recovered M.P. = 162° C.

Example 3

2-Methyl-3,4-bis(phenylmethoxy)pyridine

3A 1-(phenylmethyl)-2-methyl-3-(phenylmethoxy) 4-pyridone

73g of the product of Example 2 were dissolved in 4 liters of ethanol (dry). 25g $K_2CO_3$ were powderized and 70g benzylbromide were added. After refluxing for 4 hours and filtration, the solvent was distilled off and the residue extracted with 4 liters of hot petroleum ether in 3 parts. After filtration the petroleum ether filtrate was concentrated to 2 liters and cooled to -10°C. 31g of white crystals of the title compound of Example 3 were obtained. The formed title compound 3A was insoluble in petroleum ether.

3B 2-methyl-3,4,bis(phenylmethoxy)pyridine

To 154.8g of the product of Example 2, 86.4g benzylalcohol and 210g TPP (triphenylphosphine) suspended in 1.5 liters (dry) THF was added a solution of 139.2g diethylazodicarboxylate in 500ml tetrahydrofuran within 20 minutes. The temperature rose to 53°C for several hours. After 4.5 hours a clear solution was obtained. After standing overnight, a solution of HCl in ether (28g) was added. Hydrochlorides of Example 3 and 3A crystallized from the solution. After cooling they were isolated by filtration and washed with ether and petroleum ether. 230g of the mixture of HCl salts of 3 and 3A was suspended in 2 liters of ethyl acetate and 1 liter of water. The pH was adjusted to 7.0 with concentrated NaOH solution. The organic phase and two organic wash phases were combined and dried over $Na_2SO_4$. Flitration and distilling off the solvent yielded an oily residue which was passed through a column with 600g $SiO_2$. Ethylacetate as eluent. Only the title compound of Example 3B, 151.8g, was eluted from the column. Eluation of 3A is possible with $CH_3OH$. M.P. = 92°C. (title comp. 3)

## Example 4

### 2-Methyl-3,4-bis(phenylmethoxy)pyridine, 1-oxide

102g of the compound of Example 3B were dissolved in 200ml $CHCl_3$ and 67.5g metachloroperbenzoic acid were added while stirring in three parts. The solution was kept for three hours at 50°C then at 10 hours at room temperature. The precipitate of metachlorobenzoic acid was filtered off and the filtrate evaporated. The residue was dissolved in 300ml ethyl acetate and extracted 3 times with $Na_2CO_3$ solution ($H_2O$) each 50ml. Finally the organic phase was washed with water and dried over $Na_2SO_4$. The ethyl acetate was distilled off and the solid residue recrystallized from $CH_3OH$/water, 92.5g of the title compound as white crystals was isolated. M.P. = 106°C.

## Example 5

### 3,4-Bis(phenylmethoxy)-2-pyridinemethanol, acetate ester

3.22g of the compound of Example 4 and 20ml acetic anhydride ($Ac_2O$) were heated for one hour at 90-100°C. Excess acetic anhydride and acetic acid were distilled off and the residue dissolved in 50ml ethyl acetate and 50ml ice water. $NaHCO_3$ was added and the mixture stirred for 30 minutes. The organic layer was separated then, washed with water and dried over $Na_2SO_4$. Evaporation yielded 3.24g of the title compound. Recrystallization from $CCl_4$. M.P. = 39-47°C.

## Example 6

3,4-Bis(phenylmethoxy)-2-pyridinemethanol, acetate ester, 1-oxide

56g of the compound from Example 5 were dissolved in 250ml $CHCl_3$ and 34g metachloroperbenzoic acid were added. The temperature of the solution warmed up to 45 °C for 30 minutes. Then it was heated to 60 °C for 1 hour and then cooled to 0 °C for 1 hour. The precipitated metachlorobenzoic acid was filtered off and washed with 50ml cold $CHCl_3$. The filtrate was extracted with $NaHCO_3$ solution (ice cold). Evaporation of the organic phase yielded an oily residue. This was stirred with 100ml cold ether. 37.3g white crystals of the title compound were obtained. M.P. = 99 °C.

## Example 7

6-[(Acetyloxy)methyl]-4,5-bis(phenylmethoxy)-2-pyridinecarbonitrile

36g of the title compound of Example 6 and 12g dimethylsulfate were refluxed for 2 hours in 300ml $CH_3CN$ and then stirred for 18 hours at room temperature. The solvent was distilled off then and the oily residue containing
1-methoxy-3,4-bis(phenyl-methoxy)-2-[(acetyloxy)methyl]pyridinium monomethylsulfate was dissolved in 300ml tetrahydrofuran /$C_2H_5OH$ (8:2) and at 0 °C 6g KCN in 40ml $H_2O$ were added dropwise. After 1 additional hour of stirring the solution was evaporated (vacuo, <40 °C) and the residue dissolved in 100ml ethyl acetate and extracted 3 times with each 50ml of water. The dried organic phase was evaporated again and yielded a brown oil. This was dissolved in 500ml ether, filtered and stirred with 10g active carbon. The filtrate was cooled down then to -50 °C. 12.4g crystals of the title compound (white) were obtained after one hour. Filtration and washing with petroleum ether followed. Recrystallization yielded 10.1g. M.P. = 92 - 93 °C.

7A 2-[(Acetyloxy)methyl]-1-[[(dimethylamino)carbonyl]oxy]-3,4-bis(phenylmethoxy)pyridinium chloride

To 3.80g of the title compound of Example 6, dissolved in acetonitrile, were added 1.08g dimethylcarbamidchloride. After two hours ether was added and the crystals of the title compound were isolated by filtration. M.P. 108-109 °C dec.

7B 6-[(Acetyloxy)methyl]-4,5-bis(phenylmethoxy)-pyridinecarbonitrile

To 7g of the title compound of Example 6, dissolved in 50ml acetonitrile, were added 2.0g dimethylcarbamidchloride. After stirring, crystals from Example 7A were formed. Without isolation 2.2g trimethylsilylcyanide were added. After a short time a clear solution was obtained. Stirring was continued for 24 hours at room temperature. The solvent was distilled off then, and the residue stirred for 30 minutes with brine and ethyl acetate. The isolated organic phase was washed with water and dried. Evaporation gave an oil of crude title compound. This was purified by column chromatography on $SiO_2$, ethyl acetate/cyclohexane (6:4) as eluents. The fraction containing the title compound was collected and the solvents distilled off. The residue was dissolved in ether and cooled to -30 °C. White crystals of the title compound, 3.01g, were obtained. M.P. 98-99 °C.

## Example 8

6-(Hydroxymethyl)-4,5-bis(phenylmethoxy)-2-pyridinecarboxylic acid

37g of the title compound of Example 7 and 81.5g lithiumhydroxy hydrate in 300ml dioxane and 200ml $H_2O$ were refluxed for 60 hours. The dioxane/water was distilled off and the residue suspended in 200ml cold water, stirred for 10 minutes and filtered off, washed with 50ml ice water two times. The filter residue, white crystals, is the Li-salt of the compound of Example 8 which is very insoluble in water. It was suspended in 500ml THF and the pH was adjusted to 1.0 with concentrated HCl and a clear solution was formed. 500ml $H_2O$ were added and the title compound crystallized from the solution, 32.3g after drying over $P_2O_5$. M.P. = 197° C.

## Example 9

### 6-methyl-4,5-bis(phenylmethoxy)-2-pyridinecarbonitrile

32g of the title compound of Example 4 were dissolved in 400ml $CH_3CN$ (dry), and 13.9g dimethylsulfate were added. Heating of the solution for one hour on reflux and standing at room temperature for 24 hours gave, after evaporation, an oily residue of 1-methoxy-2-methyl-3,4-bis(phenylmethoxy)pyridinium monomethlysulfate. This was dissolved in 100ml THF/EtOH (8:2). At 0° C a solution of 7.8g KCN in 15ml $H_2O$ was added dropwise while stirring continued. Continuous stirring for one hour at room temperature and evaporation yielded a residue (solid and oil). Column chromatography of this material on silicagel EtOAc/cyclohexane (9:1) yielded 23g oily residue of the title compound of Example 1. The residue was dissolved in ether and cooled down to -5° C, 14.2g of the title compound as white crystals were obtained. M.P. = 103-105° C.

## Example 10

### 6-Methyl-4,5-bis(phenylmethoxy)-2-pyridinecarboxylic acid, methyl ester

3.30g of the title compound of Example 9 were suspended in 50ml methanol (dry), and, at 0° C, hydrochloric acid (gas) was bubbled in the suspension for 3 minutes forming a clear solution. After standing for 18 hours, the solution was evaporated and the residue dissolved in 100ml ethyl acetate/ice water (1:1). The pH was adjusted to 7 with $NaHCO_3$ solution and the organic phase separated and washed with water, dried and evaporated. The residue was dissolved in ethyl acetate and chromatographed on $SiO_2$, ethyl acetate/cyclohexane (9:1) as eluent. 1.47g pure title compound, 1.21g 6-Methyl-4,5-bis(phenylmethoxy)-2-pyridine-carboxamide, and 0.31g 1,4-Dihydro-6-methyl-4-oxo-5-(phenylmethoxy)-2-pyridinecarboxylic acid, methyl ester were isolated. M.P. = 94-95° C.

## Example 11

### 6-Methyl-4,5-bis(phenylmethoxy)-2-pyridine-carboxylic acid

3.66g of the title compound of Example 10 were dissolved in 100ml ethanol and 0.57g KOH were added after stirring for 3 hours at room temperature. A suspension of the potassium salt was formed. The solvent was distilled off then and the residue dissolved in 100ml $H_2O$. The pH was adjusted to 2 with diluted HCl. Yield: white crystals of the title compound, 3.45g after drying. M.P. = 137-139° C.

## Example 12

Alternate Preparation of 6-Methyl-4,5-bis(phenylmethoxy)-2-pyridine-carboxylic acid

3.30g of the title compound of Example 9 and 1.26g lithium hydroxyde monohydrate were dissolved in 100ml $H_2O$/THF (8:2) and refluxed for 8 hours. The THF was distilled off and the pH was adjusted to 2 with diluted HCl. Yield: 3.38g of the title compound after drying.

Example 13

6-(Hydroxymethyl)-4,5-bis(phenylmethoxy)-2-pyridinecarboxylic acid, phenylmethyl ester

0.9g of the title compound of Example 8, 0.35g $K_2CO_3$ and 0.85g benzylbromide in 10ml dimethylformamide were stirred for 24 hours at room temperature. Evaporation and crystallization of the residue yielded 0.8g of the title compound after filtration. M.P. = 93-95 °C.

Example 14

6-(Chloromethyl)-4,5-bis(phenylmethoxy)-2-pyridinecarbonylchloride Hydrochloride

10g of the title compound of Example 8 and 20ml thionylchloride were stirred for 12 hours in 250ml $CH_2Cl_2$. The solvent was distilled off then, and the residue stirred with ether, filtered and washed. Yield: 11.2g white crystals of the title compound. M.P. = 143-145 °C.

Example 15

6-(Chloromethyl)-4,5-bis(phenylmethoxy)-2-pyridinecarboxylic acid, methyl ester

11.2g of the title compound of Example 14 were stirred with 100ml methanol for 24 hours. The solvent was distilled off, and the residue dissolved in 200ml ethyl acetate/ice water. The pH was adjusted to 7 with sodiumdicarbonate, and the organic phase separated and washed with water, dried over sodium sulfate and evaporated. Yield: white crystals of the title compound, 10.1g. M.P. = 121-122 °C.

Example 16

6-(Azidomethyl)-4,5-bis(phenylmethoxy)-2-pyridinecarboxylic acid, methyl ester

2g of the title compound of Example 15 and 0.6g $NaN_3$, 0.01g potassium iodide and 0.01g 18-crown 6 were stirred in 10ml DMF overnight. The DMF was distilled off and the residue dissolved in $CHCl_3$/water. The organic phase was washed with water and dried. Evaporation gave 2.0g of the title compound, white

crystals. M.P. = 88-89 °C.

Example 17

3,4-Bis(phenylmethoxy)-2-pyridinemethanol

3.64g of the title compound of Example 5 were dissolved in 50ml $C_2H_5OH$ and 0.57 KOH were added. After stirring for three hours at room temperature, the solvent was distilled off. The residue was dissolved in 50ml water and 50ml ethyl acetate. The organic phase was separated, washed with water, dried and evaporated. Solid title compound was obtained, 3.19g. Recrystallization from cyclohexane, 3.07g. M.P. = 83.5-84.5 °C.

Example 18

2-(Chloromethyl)-3,4-bis(phenylmethoxy)pyridine

3.2g of the title compound of Example 17 were dissolved in 30ml $CH_2Cl_2$ and 1.78g thionylchloride were added. Refluxing for 15 minutes completed the reaction. Evaporation yielded a residue which was dissolved in 50ml ethyl acetate and 50ml $H_2O$. Neutralization with $NaHCO_3$ and washing of the organic phase with water, drying and distilling off the ethyl acetate yielded 2.9g of the title compound, white crystals. M.P. = 86-87 °C.

Example 19

2-(Azidomethyl)-3,4-bis(phenylmethoxy)pyridine

To 3.39g of the title compound of Example 18 in 50ml DMF and 0.1g C-18-Crown-6 were added 0.8g $NaN_3$. After heating at 80 °C for 3 hours, the solvent was distilled off in vacuo and the residue dissolved in 100ml ethyl acetate and 50ml ice water. The organic phase was separated and washed with water, dried over $Na_2SO_4$ and evaporated. Yield: 3.36g of the title compound, white crystals. Recrystallization from cyclohexane. M.P. = 45-49 °C.

Example 20

2-(Azidomethyl)-3,4-bis(phenylmethoxy)pyridine, 1-oxide

To 3.46g of the title compound of Example 19 in 50ml $CHCl_3$ were added 2g metachloroperbenzoic acid. After stirring for 24 hours the solvent was distilled off and the residue dissolved in 100ml ethyl acetate and 50ml ice water. The pH was adjusted to 7 with $Na_2CO_3$ solution and the organic phase separated, washed with water and dried over $Na_2SO_4$. Evaporation yielded 3.12g of the title compound, white crystals. M.P. = 112-114 °C.

Example 21

6-(Azidomethyl)-4,5-bis(phenylmethoxy)-2-pyridinecarbonitrile

3.75g of the title compound of Example 20 and 1.3g dimethylsulfate in 100ml $CH_3CN$ were heated under reflux for 1.5 hours. The solvent was distilled off then in vacuo and the oily residue of the title compound of Example 1 dissolved in 50ml THF/$C_2H_5OH$ (8:2). At 0°C a solution of 0.66g KCN in 5ml $H_2O$ was added dropwise within 30 minutes. After stirring for one hour, the solvents were distilled off and the residue stirred with 100ml ice water and 50ml ether. The organic phase was washed with water and evaporated. The solid residue was recrystallized from cyclohexane. Yield: white crystals of the title compound, 1.27g. M.P. = 103°C.

Example 22

6-(Azidomethyl)-4,5-bis(phenylmethoxy)-2-pyridinecarboxylic acid, methyl ester

3.71g of the title compound of Example 21 were dissolved in 100ml $CH_3OH$, and $HCL_{gas}$ was bubbled through the solution for 5 minutes. After standing for 24 hours at room temperature, the solvent was distilled off in vacuo, and the residue dissolved in ice water/ethyl acetate. The pH was adjusted to 7 with sodium bicarbonate, and the water washed. The organic phase was dried and evaporated. The residue was purified by column chromatography on silica gel, cyclohexane/ethyl acetate as eluents. 1.28g of the title compound was isolated as white crystals.

Example 23

2-(Chloromethyl)-3,4-bis(phenylmethoxy)pyridine, 1-oxide

18g of the title compound of Example 18 and 10g meta-chloroperbenzoic acid were stirred at room temperature in 100ml $CHCl_3$ for 1/2 hour. 1/2 hour additional heating was necessary to complete the reaction. The reaction solution was stirred with 200ml ice water then and $NaHCO_3$ added until $CO_2$ evolution was finished. The organic phase was washed with water then and dried. Distilling off the $CHCl_3$ yielded an oily residue which crystallized immediately, 13.5g. Recrystallization from toluene/cyclohexane (1:1) yielded 11.2g white crystals of the title compound. M.P. = 118-119°C.

Example 24

6-(Chloromethyl)-4,5-bis(phenylmethoxy)-2-pyridinecarbonitrile

2.49g of the title compound of Example 23 and 0.97g dimethylsulfate were dissolved in 40ml $CH_3CN$. Heating for 1 hour, stirring overnight at room temperature and evaporation yielded an oily residue. This was dissolved in 10ml THF/EtOH (8:2), and at 0°C 0.55g KCN in 1.5ml $H_2O$ were dropped in. Stirring was continued for one hour and the solvents were distilled off in vacuo then. The residue was suspended in 50ml ether under ice cooling. Filtration yielded a solid of 2.7g of the title compound. This was stirred with

20ml $CH_2Cl_2$ and filtered. The filtrate was concentrated and stirred with 20ml petroleum ether. The yield were white crystals of the title compound, 0.7g. M.P. = 128-130° C.

## Example 25

### 6-(chloromethyl)-4,5-bis(phenylmethoxy)-2-pyridinecarboxylic acid, methyl ester

3.64g of the title compound of Example 24 were dissolved in 50ml $CH_3OH$ and $HCl_{gas}$ was inserted for one minute at 0° C. It was left standing overnight. Evaporation yielded a residue which was dissolved in 50ml ethyl acetate/50ml ice water and neutralized with $NaHCO_3$. The organic phase was separated and washed with water and dried. The solvent was distilled off. The oily residue was chromatographed on $SiO_2$ ethyl acetate/cyclohexane (8:2). The eluent contained 1.35g of the title compound, 0.7g of 6-(chloromethyl)-1,4-dihydro-4-oxo-5-(phenylmethoxy)-2-pyridine-carboxylic acid, methyl ester, 1.4g of 6-(Chloromethyl)-4,5-bis(phenylmethoxy)-2-pyridinecarboxamide. M.P. of title compound = 141-142° C.

## Example 26

### 6-(Azidomethyl)-4,5-bis(phenylmethoxy)-2-pyridinecarboxylic acid

2g of the title compound of Example 16 and 0.4g potassium hydroxide in 50ml $C_2H_5OH$ were stirred at room temperature for two hours. A white precipitate of the potassium salt of the title compound of Example 1 was obtained. It was filtered off and dissolved in 100ml water. The pH was adjusted to 5 with 2N $H_3PO_4$ then. The title compound, 1.6g, was obtained as a precipitate (white crystals) after filtration. M.P. = 174° C.

## Example 27

### 6-(Aminomethyl)-4,5-bis(phenylmethoxy)-2-pyridinecarboxylic acid

A) 0.12g of the title compound of Example 26 dissolved in 3ml tetrahydrofuran and one drop of water were stirred and 0.08g triphenylphosphine in 2ml tetrahydrofuran were added. After stirring overnight crystals of the title compound were formed. After filtration and washing with ether the yield was 0.05g.

B) 2.9g of the title compound and 1.9g triethylamine were dissolved in 250ml $CH_2Cl_2$ and $H_2S$ was bubbled through the solution for 30 minutes. Stirring overnight at room temperature yielded 2.7g white crystals after evaporation and suspension in water. (The pH was adjusted to 5 with acetic acid and the title compound was isolated.) M.P. = 230° C dec.

## Example 28

### 6-[[[(1,1-Dimethylethoxy)carbonyl]amino]methyl]-4,5-bis(phenylmethoxy)-2-pyridinecarboxylic acid

0.6g of the title compound of Example 27, 0.69g triethylamine and 0.54g di-tert-butyldicarbonate were stirred in 60ml dioxane/10ml water at room temperature overnight. After evaporation the residue was

dissolved in 50ml $H_2O$/50ml ethyl acetate and the pH was adjusted to 2.5 with citric acid. The organic phase was washed with brine and water, dried and evaporated. The oily residue crystallized after adding 20ml petroleum ether, white crystals 0.7g of the title compound. M.P. = 144-146 °C.

## Example 29

### 6-[[(Aminocarbonyl)amino]methyl]-4,5-bis(phenylmethoxy)-2-pyridinecarboxylic acid

5g of the title compound of Example 27 were suspended in 140ml dioxane and 40ml $H_2O$. 1.25 potassium isocyanate was added and the mixture was refluxed for 4 hours. After standing overnight the pH was adjusted to 3.0 with 2N. HCl. 3.5g crystals of the title compound were formed and isolated by filtration. The dioxane of the filtrate was distilled off and crystals of a second batch of the title compound, 0.6g, were obtained. M.P. = 195-197 °C.

## Example 30

### 6-Formyl-4,5-bis(phenylmethoxy)-2-pyridinecarboxylic acid, phenylmethyl ester

0.68g of the title compound of Example 13 and 0.94g of manganese dioxide (activated) were stirred for 6 hours in 15ml $CH_2Cl_2$. After filtration over "Hyflo", the clear solution was evaporated. Yield: 0.4g white crystals of the title compound containing 1 mol $H_2O$. M.P. = 109-111 °C.

## Example 31

### 3,4-Bis(phenylmethoxy)-2,6-pyridinedicarboxylic acid, 6-(phenylmethyl) ester

0.091g of the title compound of Example 30, 0.032g $KMnO_4$ and 0.053g C18-Crown-6 in 8ml benzene were stirred overnight at room temperature, with ethyl acetate and $H_2O$. The pH was adjusted to 2.5 with 2N. $H_3PO_4$. The organic phase was washed with water, dried, stirred with active carbon and evaporated. After evaporation the residue was stirred. Yield: 0.01g of the title compound.

31A Alternative Synthesis:

### 3,4-Bis(phenylmethoxy)-2,6-pyridinedicarboxylic acid, 6-(phenylmethyl) ester

5g of the title compound of Example 30 and 5g tetrabutylammonium permanganate were dissolved in 100ml pyridine at 10 °C. After one hour the temperature was raised to 28-29 °C, and the color of the permanganate turned brown. After 1-1/2 hours no starting material was present. Then $SO_2$ gas was bubbled through the solution after it had become colorless. The pyridine was distilled off in vacuo and the residue stirred with ice water/ethyl acetate. The pH was adjusted to 2.0 with $H_3PO_4$. The organic phase was washed with water and dried over $Na_2SO_4$. Stripping off the solvent gave 4.5g of crude title compound. This material was dissolved in dioxane (75ml) at 50 °C, and then 50ml water were added. Yield: white crystals of the title compound were formed, 2.9g. M.P. = 105 °C dec.

## Example 32

### 3,4-Bis(phenylmethoxy)-2,6-pyridinedicarboxylic acid, 2-(phenylmethyl) 6-(1,1-dimethylethyl) ester

0.235mg of the title compound of Example 31, 0.04g tert-butanol and 0.148g 4-pyrrolidino-pyridine were dissolved in 20ml $CH_2Cl_2$. A solution of 0.123g dicyclohexylcarbodiimide in 5ml $CH_2Cl_2$ was added dropwise. After continuous stirring for 18 hours at room temperature, the formed dicyclohexylurea was filtered off. The solvent of the filtrate was distilled off and the residue dissolved in 20ml ethyl acetate. This was extracted 3 times with 10ml water each time and dried over $Na_2SO_4$. Evaporation and recrystallization from Ligroine gave 0.155g white crystals of the title compound. M.P. = 143 °C.

## Example 33

### 3,4-Bis(phenylmethoxy)-2,6-pyridinecarboxylic acid, 2-(1,1-dimethylethyl) ester

To 0.53g of the title compound of Example 32 in 25ml THF/$H_2O$ (9:1) were 0.053g KOH in 5ml $H_2O$ added dropwise at room temperature. After stirring for 6 hours the solvents were distilled off in vacuo. The residue was dissolved in 50ml $H_2O$/50ml ethyl acetate and the pH was adjusted to 2.5 with citric acid. The organic phase was washed and dried over $Na_2SO_4$. After evaporation 0.35g of crude title compound were obtained. This was dissolved in toluene and after adding cyclohexane, crystals of the title compound were formed, 0.25g. M.P. = 122 °C dec.

## Example 34

### 6-[(Hydroxyimino)methyl]-4,5-bis(phenylmethoxy)-2-pyridinecarboxylic acid, phenylmethyl ester

To 6g of the title compound of Example 30, dissolved in 150ml tetrahydrofuran, were added 3g isopropanol and 2.34g N,O-bistrimethylsilyl hydroxylamine. After two hours of stirring, distilling off the solvent and stirring the residue with ice water for several hours, white crystals of the title compound were formed. Yield: 6.11g after filtration and drying. M.P. = 160-163 °C.

## Example 35

### 6-Cyano-4,5-bis(phenylmethoxy)-2-pyridinecarboxylic acid, phenylmethyl ester

A) 5.7g of the title compound of Example 34 were dissolved in 300ml tetrahydrofuran. 1.7ml triethylamine were added and then 1.1ml chlorosulfonylisocyanate in 10ml tetrahydrofuran were dropped in while stirring continued. After two additional hours 10% triethylamine and 10% chlorosulfonylisocyanate were added. Stirring continued for one more hour. The solvent was distilled off and the residue dissolved in 200ml $CH_2Cl_2$ and washed with water, dilute HCl solution and water again. The organic phase was dried over $Na_2SO_4$/$MgSO_4$, filtered and evaporated. The residue of crude title compound was purified by column chromatography on $SiO_2$, eluents:
cyclohexane/tetrahydrofuran (8:2). Fr. 75-95 contained 2.4g pure title compound.

B) 6.56g of the title compound of Example 40 were suspended in 180ml $CH_3CN$ and 4.49g "diphosgen" (chloroformic acid trichloromethylester) were added dropwise. After 10 minutes a clear solution was obtained and the solvent was distilled off in vacuo. The residue was dissolved in 200ml ether and washed with water and diluted $NaHCO_3$ solution. After drying over $MgSO_4$, the organic phase was evaporated with petroleum ether. 4.8g of the title compound were obtained in pure form. M.P. = 92-94 °C.

35C Alternative Synthesis of: 6-cyano-4,5-Bis(phenylmethoxy)-2-pyridinecarboxylic acid, phenylmethylester

4.41g 4,5-Bis(phenylmethoxy)-2-pyridinecarboxylic acid, phenylmethyl ester, 1-oxide and 1.26g dimethylsulfate were refluxed for two hours in 100ml acetonitrile. The solvent was distilled off and the oily residue dissolved in 100ml tetrahydrofuran/$C_2H_5OH$ (8:2) at 0 °C. A solution of 0.66g KCN in 5ml $H_2O$ was added dropwise over a period of 30 minutes. After continuous stirring for one hour at 0-5 °C, the solvents were distilled off in vacuo and the oily residue dissolved in 100ml ethyl acetate/$H_2O$ (1:1). The organic phase was washed with water, dried and evaporated. The oily residue was purified by column chromatography on $SiO_2$, cyclohexane/tetrahydrofuran (8:2) as eluent. Fractions 85-96 contained 0.3g light yellow crystals of the title compound. The major reaction product was the title compound, of example 41 2.7g.

## Example 36

### 6-Cyano-4,5-bis(phenylmethoxy)-2-pyridinecarboxylic acid

A) 0.22g of the title compound of Example 35 dissolved in 5ml tetrahydrofuran, were stirred at -15 °C, and 0.056g KOH in 1ml $H_2O$ were added dropwise. After 30 minutes a new compound was formed. The tetrahydrofuran was evaporated after acidification with citric acid. The residue was extracted with ethyl acetate, which was dried and evaporated, 0.09g of the title compound white solid containing traces of 6-(Aminocarbonyl)-4,5-bis(phenylmethoxy)-2-pyridinecarboxylic acid.

B) Alternative preparation of 6-Cyano-4,5-bis(phenylmethoxy)-2-pyridine-carboxylic acid

0.22g of the title compound of Example 35 were stirred at 70 °C with 20ml $CH_3COOH$. After 10 hours the solution was evaporated in vacuo. The residue was extracted with ethyl acetate/$H_2O$. The pH was adjusted to 7.0 and the organic phase dried and the solvent stripped off, 0.04g of the title compound were obtained as residue.
M.P. = 167-169 °C.

## Example 37

### 6-(Aminocarbonyl)-4,5-bis(phenylmethoxy)-2-pyridinecarboxylic acid

0.18g of the title compound of Example 36 were suspended in 20ml $C_2H_5OH$. 0.04g NaOH in 1ml $H_2O$ and 0.2ml hydrogenperoxide (30%) were added. After stirring for 30 minutes, additional NaOH, 0.04g, in 1ml $H_2O$ was added. After a short time a clear solution was obtained. 55ml water were added then, and the pH was adjusted to 1.0 with 2N HCl. The precipitate of crude product was isolated by filtration and recrystallized from dioxane/water (1:1). Yield: 100mg light yellow crystals of the title compound. M.P. = 172 °C.

## Example 38

6-difluoromethyl-4,5-bis(phenylmethoxy)-2-pyridinecarboxlic acid, phenylmethylester

5.0g of the title compound of Example 30 in 50ml $CH_2Cl_2$ were treated with 1.7ml diethylaminosulfurtrifluoride and stirred for one hour at 0° C. Another 0.17ml of diethylaminosulfurtrifluoride were added, and stirring continued for one more hour. The solvent was stripped off then, and the residue dissolved in 100ml ethyl acetate and 20ml $H_2O$. $NaHCO_3$ was added, and the organic phase washed with water and dried. After distilling off the solvent and stirring the residue with 10ml ether, 3.5g white powder were obtained. Recrystallization from cyclohexane yielded 1.7g white crystals of the title compound. M.P. = 118-119° C.

## Example 39

6-difluoromethyl-4,5-bis(phenylmethoxy)-2-pyridinecarboxylic acid

To 1.8g of the title compound of Example 38, dissolved in 50ml tetrahydrofuran, a solution of 0.2 KOH in 5ml of $H_2O$ was added dropwise. After one hour the reaction was complete. The pH was adjusted with citric acid to 3.0, and the tetrahydrofuran distilled off in vacuo. The residue was dissolved in ethyl acetate/water, and the organic phase washed with water, dried and evaporated. 1.2g white solid of the title compound were obtained after stirring with petroleum ether. M.P. = 110-117° C. (dec.)

## Example 40

6-(2-cyanoethenyl)-4,5-bis(phenylmethoxy)-2-pyridinecarboxylic acid

A) Preparation of the phenylmethylester of the title compound

4.5g of the title compound of Example 30 and 3.3g triphenylcyanomethylenphosphorane were stirred at 60° C in 100ml tetrahydrofuran overnight. The solvent was distilled off. The solid residue, a mixture of the phenylmethylester and the triphenylphosphine oxide, weighed 7.8g.

B) Preparation of the title compound of Example 40

The material described in Example 40A was dissolved in 100ml tetrahydrofuran, and 1.2g KOH in 30ml $H_2O$ were added dropwise. After two hours the tetrahydrofurane was stripped off, and water was added to the suspension. A white solid was filtered off and stirred with 30ml ethyl acetate and filtered. Potassium salt of the title compound, 3.5g. This was dissolved in 100ml tetrahydrofuran/water, and the pH was adjusted to 3.0 with 2N HCl. Yield: 3.1g crystals of the title compound. M.P. = 230-233° C.

## Example 41

4,5-Bis(phenylmethoxy)-2-pyridine-carboxylic acid, phenylmethylester

21.5g (156 mmol) potassium carbonate were added to a suspension of 29.4g (120 mmol) 1,4-Dihydro-5-(phenylmethoxy)-4-oxo-2-pyridinecarboxylic acid in 350ml N,N-dimethyl-formamide and stirred for one hour at room temperature. 31ml (264 mmol) benzylbromide were added and the mixture was then heated to 100 °C under stirring for 25 hours. After cooling to room temperature, the DMF was distilled off in vacuo and the residue triturated with ethyl acetate with short heating to 60 °C. 40g inorganic salts were filtered off, the filtrate was concentrated to about 75ml and chromatographed on silica gel with ethyl acetate: petroleum ether 90:10 as eluent. Yield: 35.5g (69.6%). M.P. = 116.7 °C of title compound.

## Example 42

### 4,5-Bis(phenylmethoxy)-2-pyridinecarboxylic acid, phenylmethyl ester, 1-oxide

30g of the title compound of Example 41 and 25g (two equivalents) of metachloroperbenzoic acid were dissolved in 200ml $CHCl_3$ (dried). After 3-1/2 hours stirring at room temperature the reaction was nearly completed. The solution was cooled to -5 °C and stirred for one hour. A white precipitate was formed. This was filtered off and the filtrate was extracted three times with $NaHCO_3$ solution, washed with water and evaporated. The residue was dissolved in 200ml carbon tetrachloride and filtered. After standing overnight in a refrigerator 12.7g of the title compound crystallized out, white crystals. Concentration of the mother liquor yielded 9.8g of title compound in addition. M.P. = 91 °C dec.

## Example 43

### 6-(Acetyloxy)-4,5-bis(phenylmethoxy)-2-pyridine-carboxylic acid, phenylmethyl ester

6.25g of the title compound of Example 42 and 14g acetic acid anhydride were heated for 7 hours at 90 °C until the reaction was completed. Excess acetic acid anhydride and acetic acid were distilled off then and the residue dissolved in 100ml ethyl acetate and 100ml ice water. The pH was adjusted to 7.0 with $NaHCO_3$ and the separated organic phase washed with water, dried and evaporated. 6.15g of the title compound were obtained as a beige solid. M.P. = 106-109 °C.

## Example 44

### 1,6-Dihydro-6-oxo-4,5-(phenylmethoxy)-2-pyridinecarboxylic acid, phenylmethyl ester

6.0g of the title compound of Example 43 and 12g ammonium acetate were stirred in 100ml tetrahydrofuran/15ml water at room temperature for 28 hours. At that time chromatography indicated complete reaction. The solvent was then distilled off and the residue (oil) dissolved in 50ml $CHCl_3$ and extracted three times with 20ml $H_2O$ each time. The dried organic phase was then evaporated and the remaining oil stirred with 100ml ether. White crystals of the title compound were formed; 5.03g. M.P. = 102 °C.

## Example 45

6-Methoxy-4,5-bis(phenylmethoxy)-2-pyridinecarboxylic acid, phenylmethyl ester

5g of the title compound of Example 44 in 50ml ethanol were stirred with a solution of diazomethane in ether (150 MM $CH_2N_2$) for 24 hours. The solvent was distilled off and then the residue chromatographed on $SiO_2$ with ethyl acetate cyclohexane 1:1. Fractions 12-28 contained 0.35g title compound and fractions 30-38 1.4g,1,6-dihydro-1-methyl-6-oxo-4,5-(phenylmethoxy)-2-pyridine carboxylic acid, phenylmethyl ester each pure.

Example 45A

6-Methoxy-4,5-bis(phenylmethoxy)-2-pyridinecarboxylic acid, phenylmethyl ester

413mg of the title compound of Example 44 were dissolved in 25ml dimethylformamide. 170mg cesium carbonate were added. After stirring for one hour at room temperature, 126mg dimethylsulfate in 10ml dimethylformamide were dropped into the reaction mixture. After stirring for 4 hours, the dimethylformamide was distilled off, and the residue dissolved in ethyl acetate/$H_2O$. The organic phase was washed with water and dried over $Na_2SO_4$. Distilling off the ethyl acetate gave an oily residue, 0.5g. This was purified by column chromatography on $SiO_2$. Eluents: ethyl acetate/cyclohexane (1:1). Fractions 3-12 contained 236mg of title compound. white crystals recrystallized from petroleum ether. M.P. = 69-72° C.

Example 45B

6-Methoxy-4,5-bis(phenylmethoxy)-2-pyridinecarboxylic acid

1.26g of the title compound of Example 45 or 45A dissolved in 50ml dioxane and 0.24g KOH in 15ml $H_2O$ were stirred together for 10 hours. The solvents were distilled off and the residue dissolved in 20ml water. The pH was adjusted to 4.0 with $2NH_3PO_4$. White crystals of title compound were immediately formed. Isolation and washing with water and drying over $P_2O_5$ gave 0.69g of title compound. M.P. = 127° C.

Example 46

1,6-Dihydro-6-oxo-4,5-bis(phenylmethoxy)-2-pyridinecarboxylic acid

8.2g of the title compound of Example 43 in 100ml $C_2H_5OH$ were stirred with 1.8g KOH (3 equivalents) at room temperature for 48 hours. The formed suspension was then evaporated. The residue was dissolved in 100ml water, filtered and the pH was adjusted to 3.0 with 2N $H_3PO_4$. White crystals of title compound were formed, filtered off and washed with water and dried, 4.50g. M.P. = 215° C dec.

Example 47

4,5,6-Tris(phenylmethoxy)-2-pyridinecarboxylic acid, phenylmethyl ester

3.48g of the title compound of Example 46, 1.37g $K_2CO_3$ and 3.73g benzyl bromide in 100ml dimethylformamide were heated overnight at 90-100°C. The solvent was distilled off in vacuo then and the residue, 8.47g oil and solid, were purified by column chromatography on $SiO_2$, $CHCl_3/CH_2Cl_2$ (1:1) as eluent. Fractions 27-45 contained 1.88g of the title compound, fractions 46-62 0.73g of the title compound, and fractions 63-178 1.34g of 1,6-Dihydro-6-oxo-4,5-bis(phenylmethoxy)-1-(phenylmethyl)-2-pyridinecarboxylic acid, phenylmethyl ester, white solid. M.P. = 68-70°C of title compound.

## Example 48

### 4,5,6-Tris(phenylmethoxy)-2-pyridinecarboxylic acid

To 1.9g of the title compound of Example 47 in 100ml tetrahydrofuran were added 0.22g KOH in 10ml water. Stirring overnight at room temperature yielded a suspension of the potassium salt of the title compound. The solvent was stripped off and the residue dissolved in 200ml water and 50ml tetrahydrofuran. The pH was adjusted to 3 with 2N $H_3PO_4$. White crystals of the title compound were obtained, 1.6g. M.P. = 193°C.

## Example 49

### 6-Cyano-4,5-dihydroxy-2-pyridinecarboxylic acid

0.5g of the title compound of Example 35 were stirred in 3ml trifluoroacetic acid/0.7ml thioanisole at room temperature for 16 hours. Trifluoroacetic acid was distilled off in vacuo and the residue stirred with 10ml $CH_2Cl_2$. A precipitate of the title compound was formed and filtered off, washed with $CH_2Cl_2$ and ether. Yield: 0.15g light yellow solid of the title compound. M.P. = 220°C dec.

## Example 50

### 6-Methyl-N-(2-oxo-1-imidazolidinyl)-4,5-bis(phenylmethoxy)-2-pyridinecarboxamide

0.35g of the title compound of Example 11, 0.15g N-hydroxybenzotriazole, 0.01g 4-dimethylaminopyridine and 0.21g dicyclohexylcarbodiimide were stirred in 10ml dimethylformamide for one hour at room temperature. Then 0.1g aminoimidazolidinone was added. Stirring was continued overnight. The formed dicyclohexylurea was filtered off, and the dimethylformamide of the filtrate distilled off. The residue was recrystallized from dioxane. Yield: 0.31g of the title compound, white crystals. M.P. = 143-145°C.

## Example 51

### 6-Methoxy-4,5-bis(phenylmethoxy)-2-pyridinecarboxylic acid, 2-[(1,1-dimethylethoxy)-carbonyl]hydrazide

3.6g of the title compound of Example 45B, 0.1g N-hydroxybenzotriazole, 0.01g 4-dimethylaminopyridine, and 2.1g dicyclohexylcarbodiimide were dissolved in 100ml tetrahydrofuran. After stirring for 15 minutes at room temperature, 1.32g t-butoxycarbonylhydrazide were added. Stirring continued overnight. The formed dicyclohexylurea was filtered off then, and the filtrate evaporated. The residue was dissolved in 50ml ethyl acetate and washed with sodium carbonate solution and water. After drying over sodium sulfate, the ethyl acetate was distilled off. 3.0g of the title compound obtained as a white solid. M.P. = 70 °C.

## Example 52

### 1,4-Dihydro-5-hydroxy-6-methoxy-4-oxo-2-pyridinecarboxylic acid, hydrazide trifluoroacetate (1:1) salt

3g of the title compound of Example 51 were dissolved in 80ml methanol, and 0.5g palladium/carbon catalyst were added. A stream of hydrogen was bubbled through the reaction suspension for one hour. The catalyst was filtered off, washed with methanol, and the filtrate was evaporated. The oily residue was 1,4-Dihydro-5-hydroxy-6-methoxy-4-oxo-2-pyridinecarboxylic acid, 2-[(1,1-dimethylethoxy)carbonyl]hydrazide, 1.8g (crude). This material was stirred with 20ml trifluoroacetic acid for 30 minutes. The trifluoroacetic acid was distilled off in vacuo and the residue stirred with 100ml ether. 1.7g beige solid of title compound were obtained. M.P. = 116 °C.

## Example 53

### [1-[[[3-[[[6-Methyl-4,5-bis(phenylmethoxy)-2-pyridinyl]carbonyl]amino]-2-oxo-1-imidazolidinyl]sulfonyl]amino]-carbonyl]-2-oxo-3-azetidinyl]carbamic acid, phenylmethyl ester, monosodium salt

## Example 53A

### 6-Methyl-4,5-bis(phenylmethoxy)-2-pyridinecarboxylic acid, 1H-benzotriazol-1-yl ester

A solution of 1.75g of the title compound of Example 11, 0.75g N-hydroxybenzotriazole monohydrate, 0.01g 4-dimethylaminopyridine and 1.1g dicyclohexycarbodiimide were stirred for one hour in 30ml dimethylformamide at 5-10 °C. The formed dicyclohexylurea was filtered off and washed with 10ml dimethylformamide. The filtrate was a solution of the title compound.

## Example 53B

### 1-[(2,2-Dimethylpropylidene)amino]-2-imidazolidinone

A nearly colorless solution of 2-imidazolidinone (252 g, 2.98 mole) and 2N sulfuric acid (4 l) was cooled to 3 °C and treated with sodium nitrite (101 g, 1.46 mole) over 15 minutes not allowing the temperature to exceed 5 °C. The yellow reaction mixture was stirred at 0-5 °C for 1.5 hours after which was added zinc dust (220 g, 3.36 mole) in small portions over 35 minutes keeping the temperature below 5 °C. The zinc

dissolved completely and rapidly until reaching the equivalence point where the zinc barely dissolved. The resulting grey mixture was stirred at 0° C for 30 minutes, the cold bath was removed and stirring was continued 2 hours longer at room temperature. The mixture was passed through a pad of Celite to remove excess zinc and the clear, colorless filtrate was treated with practical grade pivaldehyde, (152.7 g, ~1.24 moles pivaldehyde). After several minutes of vigorous stirring, a colorless precipitate formed. Stirring was continued overnight (16 hours). The mixture was filtered to collect the solid and the solid washed with water (3 x 300 ml) and dried on a fluid bed dryer (45 minutes at 40° C) to yield 150 g of the title B compound as a dry freeflowing colorless powder. $R_f$ = 0.48 (silica gel, ethyl acetate:ethanol, 7:3); m.p. 184-185° C.

An analytical sample was prepared by recrystallization from chloroform: m.p. 187.3-187.7° C:

| | | | |
|---|---|---|---|
| Calc'd: | C, 56.78; | H, 8.93; | N, 24.83; |
| Found: | C, 56.54; | H, 8.94; | N, 24.68. |

## Example 53C

### (2-Oxo-1-imidazolidinyl)carbamic acid, 1,1-dimethylethyl ester

To 2N sulfuric acid (270 ml) at 80° C was added the title B compound (45 g, 0.266 mole) portionwise, over 20 minutes. During addition the pivalaldehyde distilled from the reaction pot as an azeotrope with water. When distillation was complete, the clear, colorless solution was chased with heptane (4 x 20 ml, azeotrope with water) to chase off any last traces of aldehyde. Total distillation time was 1 hour 45 minutes. The reaction was cooled to ~30° C and the pH adjusted to 7.25 with the addition of sodium hydroxide.

The aqueous solution was concentrated and the concentrate was chased with absolute ethanol to yield a nearly colorless solid. This material was suspended in methanol (360 ml), warmed to 40° C and then treated with butyloxycarbonyl-anhydride (63.8 g, 1.1 eq).

After stirring 3 hours at 40-45° C, the reaction was completed by chromatographic analysis and was filtered to remove the sodium sulfate. The filtrate was concentrated to a waxy colorless solid. This material was taken into hot acetonitrile (450 ml), seeded and allowed to crystallize at room temperature for 16 hours. It was then chilled at 5° C for 48 hours after which the solid was collected by filtration. The colorless needles were washed once with a small amount of cold acetronitrile, then twice with hexane to afford the first crop of the desired product (42.55 g). The mother liquors were concentrated and the residue taken into a minimal amount of hot acetonitrile to yield a second crop (4.78 g) for a total yield of the title C compound of 47.33 g. $R_f$ = 0.36 (silica gel, ethyl acetate: acetone 1:1); m.p. 186.4-186.5° C:

| | | | |
|---|---|---|---|
| Calc'd: | C, 47.75; | H, 7.51; | N, 20.88; |
| Found: | C, 47.64; | H, 7.47; | N, 20.92. |

## Example 53D

### [1-[[[[3-[[(1,1-Dimethylethoxy)carbonyl]amino-2-oxo-1-imidazolidinyl]sulfonyl]amino]carbonyl]-2-oxo-3-azetidinyl]-carbamic acid,phenylmethyl ester

A suspension of phenylmethyl ester 2.2 g of carbamic acid, phenylmethyl ester in 50 ml of ethyl acetate was cooled to -30° C under argon. Chloro sulfonylisocyanate (0.94 ml, 1.53 g) was added and the solution was stirred for about 45 minutes in a -25° C bath. Dichloromethane (20 ml) was added followed by the addition of the title C compound (2.2 g). Triethylamine (2.6 ml, 1.89 g) in 20 ml of methylene chloride

was added dropwise to the solution over 8 minutes (rinsed in with 3 ml of methylene chloride). The suspension was removed from the cold bath and stirred under argon for 5.5 hours. Another 0.25 ml of triethylamine was added followed by an additional 30 minutes of stirring.

The reaction mixture was poured into 100 ml of water and 36 ml of 0.5 N sulfuric acid was added. The layers were separated, the organic phase washed with 50 ml of water, dried over magnesium sulfate and concentrated in vacuo to give 5.22 g of the title D compound.

## Example 53E

[1-[[[3-[[(1,1-Dimethylethoxy)carbonyl)amino-2-oxo-1-imidazolidinyl]sulfonyl]amino]carbonyl]-2-oxo-3-azetidinyl]-carbamic acid, phenylmethyl ester, monosodium salt

The 5.22 g of foam from Example 53D was dissolved in 25 ml of acetone and dilute with 13 ml of water at a pH of about 2.3. 1.0 N aqueous NaOH was added dropwise until the pH of the solution was 6.4.

The acetone was seeded and removed slowly at room temperature (25 to 30° C bath) in vacuo to give a slurry.

The slurry was diluted with 6 ml of water containing 1 equivalent (0.01 m of NaOAc. The resultant slurry was stirred 5 to 10 minutes and then filtered to collect the solid product.

The isolated solid was washed with water (2 x 6 ml) and with ethanol (5 ml), and then the solid was dried in vacuo to give the crude product as a white solid, 4.4g (77.7 M%).

A small sample was recrystallized from aqueous acetone to give the title compound of Example 53E. The solid undergoes slow thermal decomposition about 180° C.
Anal. calc'd for $C_{20}H_{25}N_6O_9SNa$ - 1 $H_20$ (548.5/566.5):
Found: C, 42.40; H, 4.80; N. 14.83; S, 5.66; Na, 4.06; $H_20$, 3.18; C,42.51; H, 4.74; N, 14.92; S, 5.62; Na, 4.12 $H_20$, 3.13

## Example 53F

[1-[[[3-[[(1,1-Dimethylethoxy)carbonyl)amino-2-oxo-1-imidazolidinyl]sulfonyl]amino]carbonyl]-2-oxo-3-azetidinyl]-carbamic acid, phenylmethyl ester.

55g of the compound of of Example 53E were stirred with 300 ml ice water and 500 ml ethyl acetate. The pH was adjusted with cooling to 2.0 ($HCl_{conc.}$). The organic phase and the organic phase from washing the aqueous phase were washed with brine and water. dried over $Na_2SO_4$ and the solvent distilled off. Evaporation in vacuo yielded the title compound as a foam. (52g)

## Example 53G

§-[[[(3-amino-2-oxo-1-imidazolidinyl)sulfonyl]amino]carbonyl]-2-oxo-3-azetidinyl]carbamic acid, phenylmethyl ester, trifluoroacetate (1:1) salt.

26.1 g of the compound of Example 53F were distilled in 50 ml $CH_2Cl_2$ and at -5 to 0° C, 100 ml trifluoroacetic acid were added during a period of 15 minutes. After stirring for 1 hour, the solution was added to 600 ml isopropanol at 0° C. White crystals of the title compound were formed and isolated after standing for 15 minutes at -10° C. (26.9g)

Example 53H

Trimethylsilyated form of [1-[[[(3-Amino-2-oxo-1-imidazolidinyl)sulfonyl] amino]carbonyl]-2-oxo-3-azetidinyl]-carbamic acid, phenylmethyl ester

2.70g trifluoroacetic acid salt of [1-[[[(3-amino-2-oxo-1-imidazolidinyl)sulfonyl] amino] carbonyl]-2-oxo-3-azetidinyl]carbamic acid, phenylmethyl ester, were suspended in 50ml $CH_3CN$ and 2g N-methyl-N-trimethylsilyl-trifluoroacetic acid amide were added. After stirring for 30 minutes, a clear solution was obtained. Volatiles were removed in vacuo and the oily residue dissolved in 30ml tetrahydrofuran to yield a solution of the title compound 53H.

Example 53I

[1-[[[[3-[[[6-Methyl-4,5-bis(phenylmethoxy)-2-pyridinyl]carbonyl]amino]-2-oxo-1-imidazolidinyl]sulfonyl]amino]-carbonyl]-2-oxo-3-azetidinyl]carbamic acid, phenylmethyl ester,

Solutions of the title compounds of Example 53A and 53H were combined then and stirred at room temperature for 12 hours. Evaporation and stirring of the residue with 50ml ice water containing 1 drop of acetic acid for one hour, gave a precipitate of crude title compound, 3.65g, H.I. 35%.

Example 53J

[1-[[[[3-[[[6-Methyl-4,5-bis(phenylmethoxy)-2-oyridinyl]carbonyl]amino]-2-oxo-1-imidazolidinyl]sulfonvl]amino]-carbonyl]-2-oxo-3-azetidinyl]carbamic acid, phenylmethyl ester, monosodium salt

3.4g crude title compound [1-[[[[3-[[[6-Methyl-4,5-bis(phenylmethoxy)-2-pyridinyl]carbonyl]amino]-2-oxo-1-imidazolidinyl]sulfonyl]amino]carbonyl]-2oxo-3-azetidinyl]carbamic acid, phenyl-methyl ester, were dissolved in 50ml acetonitrile and sodiumbicarbonate solution (water) was added until the pH showed 6.0. Crystals of title compound were immediately formed. Yield: 1.62g of title compound white crystals after drying. M.P. = 112-116°C dec.

Example 54

[3S(Z)]-2-[[[1-(2-Amino-4-thiazolyl)-2-[[1-[[[[-3-[[(1,4-dihydro-5-hydroxy-6-methyl-4-oxo-2-pyridinyl)carbonyl]-amino]-2-oxo-1-imidazolidinyl]sulfonyl]amino]carbonyl]-2-oxo-3-azetidinyl]amino]-2-oxoethylidene] amino]oxy]-2-methylpropanoic acid, disodium salt

Example 54A

Trimethylsilyated form of N-[3-[[[(3-Amino-2-oxo-1-azetidinyl)carbonyl] amino]sulfonyl]-2-oxo-1-imidazolidinyl]-1,4-dihydro-5-hydroxy-6-methyl-2-pyridinecarboxamide monosodium salt

1.56g of the title compound of Example 53D were dissolved in 30ml dimethylformamide and 1.48ml N-methyl-N-trimethylsilyltrifluoroacetic acid amide were added. After stirring for 30 minutes, 0.5g palladium/carbon (10%) followed, and, with stirring, hydrogen was bubbled through the reaction mixture for one hour. The excess hydrogen was then expelled with nitrogen. The suspension contains in solution the title compound.

Example 54B

[3S(Z)]-2-[[[1-(2-Amino-4-thiazolyl)-2-[[1-[[[[-3-[[(1,4-dihydro-5-hydroxy-6-methyl-4-oxo-2-pyridinyl)carbonyl]-amino]-2-oxo-1-imidazolidinyl]sulfonyl]amino]carbonyl]-2-oxo-3-azetidinyl]amino]-2-oxoethylidene] amino]oxy]-2-methylpropanoic acid,diphenyl methyl ester

To the suspension of Example 54A was then added a solution of 1.22g 2-Amino-α-[[2-(diphenylmethoxy)-1,1-dimethyl-2-oxoethoxy]imino]-4-thiazoleacetic acid, 1H-benzotriazol-1-yl ester in 10ml dimethylformamide. After stirring at room temperature overnight, the catalyst was removed by filtration, washed with 15ml dimethylformamide, and the dimethylformamide of the combined filtrates was distilled off (<40° C). The oily residue was stirred with ice water (100ml) and a few drops of acetic acid for one hour. Crude title compound, 1.6g, was obtained as a beige solid.

Example 54C

[3S(Z)]-2-[[[1-(2-Amino-4-thiazolyl)-2-[[1-[[[[-3-[[(1,4-dihydro-5-hydroxy-6-methyl-4-oxo-2-oyridinyl)carbonyl]-amino]-2-oxo-1-imidazolidinyl]sulfonyl]amino]carbonyl]-2-oxo-3-azetidinyl]amino]-2-oxoethylidene] amino]oxy]-2-methylpropanoic acid, disodium salt

1.5g crude title compound of Example 54B were stirred in 20ml trifluoroacetic acid and 5ml anisole at -10° C for 30 minutes. Crude title compound was obtained as the TFA-salt of the free acid by precipitation with diethylether, 1.26g.

1.20g of this material were suspended in 10ml water, and the pH was adjusted to 5.5 with sodium bicarbonate. The clear, formed solution was passed through an Organogen column (30cm; 0.8mm), water as eluent. Fractions 75-105 (each 15ml) contained purified title compound, 0.21g. M.P. = >290° C dec.

Example 55

[1-[[[[3-[[[6-Methoxy-4,5-bis(phenylmethoxy)-2-pyridinyl]carbonyl]amino]-2-oxo-1-imidazolidinyl]sulfonyl]amino]-carbonyl]-2-oxo-3-azetidinyl]carbamic acid, phenylmethyl ester, monosodium salt

Example 55A

Hydroxybenzotriazole ester of Example 45B

7.0g of the title compound of Example 45B, 2.87g N-hydroxybenzotriazole, 0.1g 4-dimethylaminopyridine and 3.9g dicyclohexylcarbodiimide were dissolved in 250ml dimethylformamide and stirred for 2 hours at room temperature. The dicyclohexylurea formed was filtered off. The filtrate was solution A. This was the hydroxybenzotriazole ester of Example 45B.

10.3g trifluoroacetic acid-salt of the title compound of Example 53H and 15ml N-methyl-N-trimethylsilyl-trifluoroacetic acid amide were stirred for 1 hour at room temperature. The solvent and the formed $CF_3COOSi \leq$ were then distilled off in vacuo and the residue dissolved in DMF and stirred overnight with the solution of Example 55a. The solvent was then distilled off in vacuo and the residue stirred for two hours in ice water with 3 drops of acetic acid. A white solid was obtained; filtered off and washed with water. 17g of this crude material was dissolved in acetonitrile and concentrated. Sodium bicarbonate solution (water) was added until the pH became 6.0. Some water was added and crystals of the title compound were formed, 4.3g. M.P. = 159-162° C dec.

## Example 56

[3S(Z)]-2-[[[1-(2-Amino-4-thiazolyl)-2-[[1-[[[[3-[[(1,4-dihydro-5-hydroxy-6-methoxy-4-oxo-2-pyridinyl)carbonyl]-amino]-2-oxo-1-imidazolidinyl]sulfonyl]amino]carbonyl]-2-oxo-3-azetidinyl]amino]-2-oxoethylidene]amino]oxy]-2-methyl-propanoic acid, disodium salt

## Example 56A

N-[3-[[[(3-Amino-2-oxo-1-azetidinyl)carbonyl]amino]sulfonyl]-2-oxo-1-imidazolidinyl]-1,4-dihydro-5-hydroxy-6-methoxy-2-pyridine-carboxamide

2.3g of the title compound of Example 55 were dissolved in 50ml dimethylformamide and 3.3g N-methyl-N-trimethylsilyl-trifluoroacetic acid amide, and 0.5g palladium/carbon (10%) were added. A stream of hydrogen was bubbled through the reaction mixture for one hour. The catalyst was filtered off then and washed three times with 10ml dimethylformamide each time. The filtrate and the washing filtrate was a solution of compound 56A.

## Example 56B

[3S(Z)]-2-[[[1-(2-Amino-4-thiazolyl)-2-[[1-[[[[3-[[(1,4-dihydro-5-hydroxy-6-methoxy-4-oxo-2-pyrdinyl)carbonyl]-amino]-2-oxo-1-imidazolidinyl]sulfonyl]amino]carbonyl]-2-oxo-3-azetidinyl]amino]-2-oxoethylidene]amino]oxy]-2-methyl-propanoic acid, disodium salt

To the combined filtrate and the washing filtrate of Example 56A were added 1.6g 2-Amino-α-[[2-diphenylmethoxy)-1,1-dimethyl-2-oxoethoxy]imino]-4-thiazoleacetic acid, IH-benzotriazol-1-yl ester. The solution was stirred for 12 hours at room temperature. The dimethylformamide was distilled off then and the residue stirred with 100ml ice water and two drops acetic acid for one hour. A beige solid was formed; 3.4g crude diphenyl methyl ester of the free acid of the title compound Example 56 after filtration and drying. 3.3g of crude diphenyl methyl ester of the free acid of the title compound Example 56A were stirred for 30 minutes with 50ml trifluoroacetic acid/anisole at 0° C. Then 150ml ether were added with cooling and the precipitate, the trifluoroacetic acid salt of crude free acid title compound was filtered off and washed with

50ml isopropanol and dried; 1.1g beige solid.

This material was suspended in 30ml water and the pH was adjusted to 6.0 with sodium bicarbonate. After filtration the solution was freeze-dried. Yield: 0.9g solid, crude title compound of Example 56. Purification of this material by column chromatography on a reverse phase organogen column, water as eluent, yielded 0.23g title compound in fractions 80-124 (3ml each). M.P. = 375 °C dec.

## Example 57

[1-[[[[3-[[[6-(Chloromethyl)-4,5-bis(phenylmethoxy)-2-pyridinyl]carbonyl]amino]-2-oxo-1-imidazolidinyl]sulfonyl]-amino]carbonyl]-2-oxo-3-azetidinyl]carbamic acid, phenylmethyl ester, monosodium salt

10.9g trifluoroacetate salt of the title compound of Example 53H (freshly prepared) were suspended in 130ml acetonitrile and 22.4ml N-methyl-N-trimethylsilyl-trifluoroacetic acid amide were added. A clear solution was formed which was stirred for one hour. The solvent and formed $CF_3COOSi$- were distilled off then in vacuo. The residue was dissolved in 50ml tetrahydrofuran (dry) and 7.8ml N-methyl-N-trimethylsilyl-trifluoroacetic acid amide were added followed by 8.5g of the title compound of Example 14 in 50ml tetrahydrofuran. Stirring overnight at room temperature followed by evaporation and stirring the residue with 200ml ice water and two drops acetic acid for 1 hour gave 19.9g crude title compound free acid. It was dissolved in 100ml acetonitrile and 4g sodiumbicarbonate, in minimum amount of water, were added. 10.7g crystals of title compound were obtained after standing for several hours at 0 °C. M.P. = 206-208 °C.

## Example 58

[1-[[[[3-[[[6-(Chloromethyl)-4,5-bis(phenylmethoxy)-2-pyridinyl]carbonyl]amino-2-oxo-1-imidazolidinyl]sulfonyl]-amino]-2-oxo-3-azetidinyl]carbamic acid, phenylmethyl ester

10.6g of the title compound of Example 57 in 200ml water and 400ml ethyl acetate were stirred at 0 °C and the pH was adjusted to 2 with 2N HCl. Organic phase was separated and the water phase extracted again with 200ml ethyl acetate. The combined organic phases were washed with brine, dried and evaporated in vacuo yielding 7.5g of the title compound as foam. M.P. = 128-130 °C.

## Example 59

[3S(Z)]-2-[[[1-(2-Amino-4-thiazolyl)-2-[[1-[[[[3-[[[1,4-dihydro-5-hydroxy-4-oxo-6-(pyridiniomethyl)-2-oyridinyl]-carbonyl]amino]-2-oxo-1-imidazolidinyl]sulfonyl]amino]carbonyl]-2-oxo-3-azetidinyl]amino]-2-oxoethylidene]-amino]oxy]-2-methylpropanoic acid, monosodium salt

## Example 59A

Preparation of [1-[[[[3-[[[4,5-Bis(phenylmethoxy)-6-(pyridiniomethyl)-2-pyridinyl]carbonyl]amino]-2-oxo-1-imidazolidinyl]sulfonyl]amino]carbonyl]-1-oxo-3-azetidinyl]carbamic acid, phenylmethyl ester, chloride

To 3.17g of the title compound of Example 58 in 50ml dimethylformamide dissolved were added 0.001g KI and 0.01 C-18-Crown 6 and 1.27g pyridine. After stirring for 6 hours additional 3ml pyridine were added and stirring continued for 12 hours. The solvent was distilled off below 50°C in vacuo and the residue was stirred with 20ml isopropanol. A beige solid was obtained, crude title compound 59A.

## Example 59B

Preparation of [1-[[[[3-[[[4,5-Bis(phenylmethoxy)-6-(pyridiniomethyl)-2-pyridinyl]carbonyl]amino]-2-oxo-1-imidazolidinyl]sulfonyl]amino]carbonyl]-1-oxo-3-azetidinyl]carbamic acid, phenylmethyl ester, inner salt

Crude product from Example 59A was stirred three times each with 20ml ice water and 1 drop acetic acid. After filtration and washing with water 3g solid material (C1 negative) were obtained; of title compound.

## Example 59C

Preparation of N-[3-[[[(3-Amino-2-oxo-1-azetidinyl)carbonyl]amino]sulfonyl]-2-oxo-1-imidazolidinyl]-1,4-dihydro-5-hydroxy-6-(pyridiniomethyl)-2-pyridinecarboxamide, trifluoroacetate (1:3) salt

3.3g of the product of Example 59B were stirred in a mixture of 15ml trifluoroacetic acid and 3ml thioanisole at room temperature overnight. 50ml isopropanol were then added and 1.8g solid title compound were obtained and filtration and washing with water.

## Example 59D

Preparation of [3S(Z)]-2-[[[1-(2-Amino-4-thiazolyl)-2-[[1-[[[[3-[[[1,4-dihydro-5-hydroxy-4-oxo-6-(pyridiniomethyl)-2-pyridinyl]carbonyl]amino]-2-oxo-1-imidazolidinyl]sulfonyl]amino]carbonyl]-2-oxo-3-azetidinyl]amino]-2-oxoethylidene]amino]oxy]-2-methylpropanoic acid, diphenylmethylester

1.8g of the product of Example 59C were suspended in 30ml acetonitrile and 3.1ml N-methyl-N-trimethylsilyl-trifluoroacetic acid amide were added. Stirring for 30 minutes yielded a clear solution after 45 minutes. The solvent and formed trimethylsilyltrifluoroacetate were distilled off in vacuo and the residue dissolved in 60ml tetrahydrofuran and 10ml dimethylformamide and 1.7g (Z)-2-Amino-<a-[[2-(diphenylmethoxy)-1,1-dimethyl-2-oxoethoxy]imino]-4-thiazoleacetic acid, 1H-benzotriazol-1-yl ester were added. After stirring for 48 hours a clear solution was obtained. Stripping off the solvents in vacuo yielded a residue which was stirred with 100ml ice water at pH 3.5. The solid was filtered off and stirred again with 50ml ice water at pH 5. (2n$H_3PO_4$) After filtration and drying 2.4g 2N $H_3PO_4$ of crude material was obtained. This material was stirred with 30ml ethanol and filtered, 1.8g of title compound was obtained.

## Example 59E

[3S(Z)]-2-[[[1-(2-Amino-4-thiazolyl)-2-[[1-[[[[3-[[[1,4-dihydro-5-hydroxy-4-oxo-6-(pyridiniomethyl)-2-pyridinyl]-carbonyl]amino]-2-oxo-1-imidazolidinyl]sulfonyl]amino]carbonyl]-2-oxo-3-azetidinyl]amino]-2-oxoethylidene]-amino]oxy]-2-methylpropanoic acid, trifluoroacetate

0.9g of the product of Example 59D were suspended in 2ml anisole and 10ml trifluoroacetic acid were added dropwise while stirring at 0° C. After 1 hour 100ml ether were added dropwise and 0.8g of crude title compound were formed as a precipitate.

## Example 59F

Preparation of [3S(Z)]-2-[[[1-(2-Amino-4-thiazolyl)-2-[[1-[[[[3-[[[1,4-dihydro-5-hydroxy-4-oxo-6-(pyridiniomethyl)-2-pyridinyl]carbonyl]amino]-2-oxo-1-imidazolidinyl]sulfonyl]amino]carbonyl]-2-oxo-3-azetidinyl]amino]-2-oxoethylidene]amino]oxy]-2-methylpropanoic acid, monosodium salt

0.75g of the product of Example 59E were suspended in 20ml water and the pH was adjusted to 6.0 with sodium bicarbonate. After filtration the filtrate was freeze-dried. 0.65g solid, crude material was obtained. It was dissolved in 10ml water and 2ml acetone and passed through a reverse phase organogen column, acetone/water gradient from 6:1 to 3:7. Seven fraction collections contained title compound; 128mg. This material was chromatographed on XAD with water as eluent first and then water/acetone (8:2). Fractions 80-88 contained 60mg title compound. M.P. = 255° C dec.

## Example 60

[1-[[[[3-[[[6-[[[(1,1-Dimethylethoxy)carbonyl]amino]methyl]-4,5-bis(phenylmethoxy)-2-pyridinyl]carbonyl]amino]-2-oxo-1-imidazolidinyl]sulfonyl]amino]carbonyl]-2-oxo-3-azetidinyl]carbamic acid, phenylmethyl ester, monosodium salt

Solution A: 3.0g of the title compound of Example 28 in 60ml tetrahydrofuran and 0.98g HOBT and 1.32g dicyclohexylcarbodiimide were stirred for 1 hour at room temperature. The formed dicyclohexylurea was filtered off; filtrate = solution A.

Solution B: 3.5g of the trifluroacetic acid salt of the title compound of Example 53H (freshly prepared) and 7.2ml N-methyl-N-trimethylsilyltrifluoroacetic acid amide were dissolved in 40ml acetonitrile during one hour. Evaporation in vacuo and redissolving of the oily residue in 30ml tetrahydrofuran and 1ml N-methyl-N-trimethylsilyltrifluoroacetic acid amide yielded solution B.

Both solutions were stirred together overnight at room temperature. After distilling off the solvent the residue was stirred with 100ml ice water and 1 drop of acetic acid for 1 hour. 5.9 crude free acid title compound were isolated as solid. This material was dissolved in 30ml acetonitrile and the pH was adjusted to 6.0 with saturated sodium bicarbonate solution. Water was added until crystals of title compound began to separate. One hour of standing at 5° C gave 4.3g crystals of title compound. M.P. = 165-168° C.

## Example 61

[1-[[[[3-[[[6-[[[(1,1-Dimethylethoxy)carbonyl]amino]methyl]-4,5-bis(phenylmethoxy)-2-pyridinyl]carbonyl]amino-2-oxo-1-imidazolidinyl]sulfonyl]amino]carbonyl]-2-oxo-3-azetidinyl]carbamic acid

4.3g of the title compound of Example 60 were suspended in 200ml water/ethyl acetate (1:1), and under ice cooling the pH was adjusted to 2.5. The organic phase was separated, washed with water, dried and evaporated. The residue, after stirring with petroleum ether, gave 4.0g white solid title compound. M.P. =

132-135 °C.

Example 62

[1-[[[[3-[[[6-[[(Aminocarbonyl)amino]methyl]-4,5-bis(phenylmethoxy)-2-pyridinyl]carbonyl]amino]-2-oxo-1-imidazolidinyl]sulfonyl]amino]carbonyl]-2-oxo-3-azetidinyl]carbamic acid, phenylmethyl ester, monosodium salt

Example 62A

[1-[[[[3-[[[6-[[Amino]methyl]-4,5-bis(phenylmethoxy)-2-pyridinyl]carbonyl]amino]-2-oxo-1-imidazolidinyl]sulfonyl]-amino]carbonyl]-2-oxo-3-azetidinyl]carbamic acid -trifluoroacetic acid salt

To 3.06g of the title compound of Example 61 in 1.5ml methylene chloride and 5ml anisole were added 20ml trifluoroacetic acid dropwise at -5 °C. After stirring for one hour at 0-5 °C the trifluoroacetic acid was stripped off in vacuo and 80ml isopropanol/ether (1:1) were added to the residue. The TFA salt of the deprotected base of 61 was obtained as a white solid 2.45g.

Example 62B

[1-[[[[3-[[[6-[[(Aminocarbonyl)amino]methyl]-4,5-bis(phenylmethoxy)-2-pyridinyl]carbonyl]amino]-2-oxo-1-imidazolidinyl]sulfonyl]amino]carbonyl]-2-oxo-3-azetidinyl]carbamic acid, phenylmethyl ester, monosodium salt

2.2g of the title compound of Example 62A were suspended in 100ml acetonitrile and 1ml N-methyl-N-trimethylsilyl-trifluoroacetic acid amide was added. After stirring for 1 hour at room temperature and evaporation the oily residue was dissolved in 100ml tetrahydrofuran, and at 0 °C 0.2ml chlorocarbonylisocyanate in 20ml tetrahydrofurane were added within 30 minutes. After 3 hours stirring the solvent was stripped off and the solid residue stirred with 50ml ice water and one drop of acetic acid for 2 hours. The precipitate was filtered off, 2.25g, after drying, title compound free acid. It was dissolved in acetonitrile and the pH was adjusted to 6.0 with sodium bicarbonate solution (water). 0.1g crystals were formed and filtered off. To the filtrate was added ether until crystallization started. After standing for 1 hour 1.7g of title compound were formed and isolated by filtration. M.P. = 210 °C.

Example 63 (Alternative of Example 62)

[1-[[[[3-[[[6-[[(Aminocarbonyl)amino]methyl]-4,5-bis(phenylmethoxy)-2-pyridinyl]carbonyl]amino]-2-oxo-1-imidazolidinyl]sulfonyl]amino]carbonyl]-2-oxo-3-azetidinyl]carbamic acid, phenylmethyl ester, monosodium salt

To 4.1g of the title compound of Example 29 dissolved in 80ml tetrahydrofuran and 50ml dimethylformamide, were added 1.52g HOBT and 0.1g 4-dimethylaminopyridine. Then a solution of 2.1g dicyclohexylcarbodiimide in 20ml tetrahydrofuran was added. After two hours the formed dicyclohexylurea was filtered off. The filtrate was solution A. 5.4g trifluoroacetic acid salt of the title compound of Example 53H were suspended in 50ml acetonitrile and 9.3ml N-methyl-N-trimethylsilyl-trifluoroacetic acid amide were added.

After stirring for 1 hour at room temperature, a clear solution was obtained.

The solvent and formed trifluoroacetic acid trimethylsilylester was distilled off in vacuo. The residue was dissolved in 50ml acetonitrile. This was solution B.

Solutions A and B were then stirred together for 12 hours at room temperature. The solvents were then distilled off in vacuo, and the residue stirred with ice water and 3 drops of acetic acid until a white solid of crude material was formed, log. This material was dissolved in 40ml acetonitrile and sodium bicarbonate (water) solution was added until the pH showed 5.5. Water was added until crystals of title compound were formed. After standing for 1 hour in the cold, 6g of title compound were isolated by filtration. M.P. = 178° C dec.

Example 64

[3S(Z)]-2-[[[2-[[1-[[[[3-[[[6-[[(Aminocarbonyl)amino]methyl]-1,4-dihydro-5-hydroxy-4-oxo-2-pyridinyl]carbonyl]-amino]-2-oxo-1-imidazolidinyl]-sulfonyl]amino]carbonyl]-2-oxo-3-azetidinyl]amino]-1-(2-amino-4-thiazolyl)-2-oxoethylidene]amino]oxy]-2-methylpropanoic acid, disodium salt

Example 64A

[3S(Z)]-2-[[[2-[[1-[[[[3-[[[6-[[(Aminocarbonyl)amino]methyl]-1,4-dihydro-5-hydroxy-4-oxo-2-pyridinyl]carbonyl]-amino]-2-oxo-1-imidazolidinyl]-sulfonyl]amino]carbonyl]-2-oxo-3-azetidinyl]amino]-1-(2-amino-4-thiazolyl)-2-oxoethylidene]amino]oxy]-2-methylpropanoic acid, diphenylmethylester

1.70g of the title compound of Example 63 and 1.12ml N-methyl-N-trimethylsilyl-trifluoroacetic acid amide were dissolved in 20ml dimethylformamide and 0.9g palladium/carbon (10%) were added. After hydrogenation for one hour (1atm.) at room temperature, the catalyst was filtered off and washed with 20ml dimethylformamide. The filtrate contained a solution of 6-[[(aminocarbonyl)amino]methyl]-N-[3-[[[(3-amino-2-oxo-1-azetidinyl)carbonyl]amino]sulfonyl]-2-oxo-1-imidazolidinyl]-1,4-dihydro-5-hydroxy-4-oxo-2-pyridinecarboxamide in the form of the silylated monosodium salt. To this was added 1.11g of the activated ester 2-amino-α-[[2-(diphenylmethoxy)-1,1-dimethyl-2-oxoethoxy]imino]-4-thiazolacetic acid, 1H-benzotriazol-1-yl ester and stirring for 16 hours at room temperature completed the reaction. The dimethylformamide was distilled off in vacuo and the residue stirred with one drop acetic acid in 50ml ice water for one hour. Yield: 1.7g crude of the title compound after drying.

Preparation and Purification of the Title Compound of Example 64

1.6g crude of the title compound of Example 64A were suspended in 4ml anisole and 20ml trifluoroacetic acid were added dropwise at -10° C. After one hour of stirring 150ml ether were added forming a precipitate of the free acid of the title compound trifluoroacetic acid salt (crude), 1.2g after drying. This material was suspended in 50ml water and the pH was adjusted to 6.0. The clear solution was refrigerated. Yield: crude title compound. Purification was accomplished by column chromatography. M.P. = 270° C dec.

Example 65

[3S(Z)]-2-[[[1-(2-Amino-4-thiazolyl)-2-[[1-[[[-[3-[[[1,4-dihydro-5-hydroxy-6-(hydroxymethyl)-4-oxo-2-pyridinyl]-

carbonyl]amino]-2-oxo-1-imidazolidinyl]sulfonvl]amino]carbonyl]-2-oxo-3-azetidinyl]amino]-2-oxoethylidene]-amino]oxy]propanoic acid, disodium salt

## Example 65A

[3-[[[3-[[[6-(Hydroxymethyl)-4,5-bis(phenylmethoxy)-2-pyridinyl]carbonyl]amino]-2-oxo-1-imidazolidinyl]sulfonyl]-amino]carbonyl]-2-oxo-3-azetidinyl]carbamic acid, phenylmethyl ester, monosodium salt

1.9g trifluoroacetic acid salt of the title compound of Example 53H and 2.5ml N-methyl-N-trimethylsilyl-trifluoroacetic acid amide were dissolved in 100ml acetonitrile during one hour of stirring at room temperature. The solvent and formed trimethylsilyltrifluoroacetate were then distilled off in vacuo. The oily residue was dissolved in 20ml dimethylformamide and 1.4g of the title compound of Example 8, 0.6g N-hydroxybenzotriazole were added. A solution of 1g dicyclohexylcarbodiimide in 10ml dimethylformamide was added dropwise and stirring continued for 16 hours. The formed dicyclohexylurea was filtered off (0.7g) and the solvent distilled off (40° C(oil vacuo). The oily residue was stirred with 100ml ice water and one drop of acetic acid. The formed white solid was filtered off, washed with water and dried, 3.5g. This material was dissolved in 50ml ethyl acetate and the pH was adjusted to 6.5 with sodium bicarbonate solution (water). 1g white solid was formed and isolated by filtration and dried, to yield the title compound.

## Example 65B

[3S(Z)]-2-[[[1-(2-Amino-4-thiazolyl)-2-[[1-[[[-[3-[[[1,4-dihydro-5-hydroxy-6-(hydroxymethyl)-4-oxo-2-pyridinyl]-carbonyl]amino]-2-oxo-1-imidazolidinyl]sulfonyl]amino]carbonyl]-2-oxo-3-azetidinyl]amino]-2-oxoethylidene]-amino]oxy]propanoic acid, disodium salt

1g of the title compound of Example 65A and 0.7g N-methyl-N-trimethylsilyl-trifluoroacetic acid amide and 0.4g palladium/carbon (10%) were hydrogenated for one hour at room temperature in 20ml dimethylformamide. The catalyst was filtered off then and washed with 20ml dimethylformamide. To the combined filtrates were added 0.7g 2-Amino-α-[[2-(diphenylmethoxy)-1,1-dimethyl-2-oxoethoxy]imino]-4-thiazolacetic acid, 1H-benzotriazol-1-yl ester. After stirring for 24 hours the solvent was distilled off and the residue stirred with 50ml ice water and one drop acetic acid for 1 hcur. A beige precipitate was formed, 1g after drying, [3S(Z)]-2-[[[1-(2-Amino-4-thiazolyl)-2-[[1-[[[-[3-[[[1,4-dihydro-5-hydroxy-6-(hydroxymethyl)-4-oxo-2-pyridinyl]carbonyl]amino]-2-oxo-1-imidazolidinyl]sulfonyl]amino]carbonyl]-2-oxo-3-azetidinyl]amino]-2-oxoethylidene]-amino]oxy]propanoic acid, diphenylmethylester. This material was stirred at 0° C with 25ml trifluoroacetic acid/anisole (4:1) for one hour. 100ml ether were added and the formed trifluoroacetic acid salt of the free acid of the title compound was isolated as a beige solid, 0.75g. This was suspended in 30ml water and the pH was adjusted to 6.0 with sodium bicarbonate. The clear solution was freeze dried, yielding 0.8g crude material of the title compound, which was purified by column chromatography. M.P. = >280° C dec.

## Example 66

[3-[[[3-[[[4,5,6-Tris(phenylmethoxy)-2-pyridinyl]carbonyl]amino]-2-oxo-1-imidazolidinyl]sulfonyl]amino]carbonyl]-2-oxo-3-azetidinyl]-carbamic acid, phenylmethyl ester, monosodium salt

Solution A: 1.9g of the title compound of Example 8, 0.65g HOBT, and 0.89g dicyclohexylcarbodiimide were stirred in 40ml tetrahydrofuran at 0° C for one hour. The formed dicyclohexylurea was filtered off and washed with tetrahydrofurane. Filtrate = solution A.

Solution B: 2.4g trifluoroacetic acid salt of the title compound of Example 53H and 4ml N-methyl-N-trimethylsilyl-trifluoroacetic acid amide were stirred for one hour in 30ml acetonitrile. The solvent and formed trimethylsilyltrifluoroacetate were stripped off in vacuo then. The residue was dissolved in 30ml tetrahydrofuran (dry) and 1.5ml N-methyl-N-trimethylsilyl-trifluoroacetic acid amide were added. After stirring for 30 minutes solution B was formed.

Solution A and B were then stirred together at room temperature for 12 hours. Evaporation and stirring for one hour with 100ml ice water containing one drop of acetic acid yielded 4.8g crude free acid of title compound. This was dissolved in 50ml acetonitrile. The pH was adjusted to 6.0 with saturated sodium bicarbonate solution. 1.8g crystals of title compound were obtained after standing for one hour at 5° C. M.P. = 175° C.

## Example 67

2-[[[1-(2-Amino-4-thiazolyl)-2-[[1-[[[[3-[[(1,4-dihydro-5,6-dihydroxy-4-oxo-2-oyridinyl]carbonyl]amino]-2-oxo-1-imidazolidinyl]sulfonyl]amino]carbonyl]-2-oxo-3-azetidinyl]amino]-2-oxo-ethylidene]amino]oxy]-2-methylpropanoic acid, disodium salt

## Example 67A

2-[[[1-(2-Amino-4-thiazolyl)-2-[[1-[[[[3-[[(1,4-dihydro-5,6-dihydroxy-4-oxo-2-pyridinyl]carbonyl]amino]-2-oxo-1-imidazolidinyl]sulfonyl]amino]carbonyl]-2-oxo-3-azetidinyl]amino]-2-oxo-ethylidene]amino]oxy]-2-methylpropanoic acid, diphenylmethylester

1.74g of the title compound of Example 66 and 0.37ml N-methyl-N-trimethylsilyl-trifluoroacetic acid amide were dissolved in 30ml dimethylformamide. 0.8g palladium/carbon (10%) were added, and for 1-1/2 hours a stream of hydrogen was bubbled through the suspension. Then 1.34g 2-amino-α-[[2-(diphenylmethoxy)-1,1-dimethyl-2-oxoethoxy]imino]-4-thiazolacetic acid, 1H-benzotriazol-1-yl ester were added and stirring was continued for 16 hours. The catalyst was then filtered off, washed with dimethylformamide and the filtrate solvent distilled off in vacuo. The residue was stirred in 50ml ice water and two drops of acetic acid for 1 hour. A solid of crude benzhydryl ester of title compound was formed and isolated by filtration, 1.7g. = title compound 67A.

This material was stirred in a mixture of 20ml trifluoroacetic acid and 4ml anisole at 0° C for 30 minutes. After adding 100ml ether, the trifluoroacetic acid salt of the free acid of Example 67A was isolated as a precipitate. This was suspended in 20ml ice water. The pH was adjusted to point 6 with sodium bicarbonate and the formed solution was passed through a reverse phase column (organogen), water as an eluente. Fractions 37-42 contained 0.16g and fractions 50-65 contained 0.33g of the title compound. = Example 67. M.P. = 50° (dec.)

## Example 68

[1-[[[3-[[[6-(Aminocarbonyl)-4,5-bis(phenylmethoxy)-2-pyridinyl]carbonyl]amino]-2-oxo-1-imidazolidinyl]sulfonyl]-amino]carbonyl]-2-oxo-3-azetidinyl]carbamic acid, phenylmethyl ester, monosodium salt

1.21g of the title compound of Example 37, 0.49g HOBT, 0.001g 4-dimethylaminopyridine and 0.66g dicyclohexylcarbodiimide were stirred in 40ml tetrahydrofuran and dried at room temperature for 1 hour. The formed dicyclohexylurea was filtered off then and washed with 10ml tetrahydrofuran. To the combined filtrate was added a solution of the silylated form of Example 53H (obtained from 1.92g trifluoroacetic acid salt of Example 53H and 3.6ml N-methyl-N-trimethylsilyl-trifluoroacetic acid amide in 20ml acetonitrile by stirring for one hour, evaporation and redissolving the oily residue in 20ml acetonitrile.

After stirring overnight the solvent was distilled off and the residue stirred with ice water and three drops of acetic acid for one hour. A precipitate of 3.7g crude [1-[[[[3-[[[6-(Aminocarbonyl)-4,5-bis(phenylmethoxy)-2-pyridinyl]carbonyl]amino]-2-oxo-1-imidazolidinyl]sulfonyl]amino]carbonyl]-2-oxo-3-azetidinyl]-carbamic acid, phenylmethyl ester was obtained.

This material was dissolved in 35ml acetonitrile and filtered. To the filtrate was added sodiumbicarbonate solution until the pH reached 6.0. Crystals of title compound, I.lg, were formed and isolated by filtration. M.P. = 190-195 °C.

## Example 69

**[3S(Z)]-2-[[[2-[[1-[[[[3-[[[6-(Aminocarbonyl)-1,4-dihydro-5-hydroxy-4-oxo-2-pyridinyl)carbonyl]amino]-2-oxo-1-imidazolidinyl] sulfonyl]amino]carbonyl-2-oxo-3-azetidinyl]amino]-1-(2-amino-4-thiazolyl)-2-oxoethylidene]amino]-oxy]-2-methylpropanoic acid, disodium salt**

1.13g of the title compound of Example 68 and 0.8ml N-methyl-N-trimethylsilyl-trifluoroacetic acid amide were dissolved in 20ml dimethylformamide. 0.5g palladium/carbon (10%) were added. After hydrogenation for 1 hour and filtration 0.78g of 2-Amino-(α-[[2-(diphenylmethoxy)-1,1-dimethyl-2-oxoethoxy]-imino]-4-thiazolacetic acid, 1H-benzotriazol-1-yl ester were added to the filtrate. Continuous stirring for 18 hours and distilling off the solvent gave a residue which was stirred for 1 hour in ice water at pH 3 (acetic acid). A precipitate of crude [3S(Z)]-2-[[[2-[[1-[[[[3-[[[6-(Aminocarbonyl)-1,4-dihydro-5-hydroxy-4-oxo-2-pyridinyl)carbonyl]amino]-2-oxo-1-imidazolidinyl]sulfonyl]amino]carbonyl]-2-oxo-3-azetidinyl]amino]-1-(2-amino-4-thiazolyl)-2-oxo-ethylidene]amino]oxy]-2-methylpropanoic acid, diphenylmethylester, 1.1g, was obtained.

The material was suspended in 3ml anisole. At -10 °C 20ml trifluoroacetic acid were added while stirring continued. Adding ether after 30 minutes gave a precipitate of the crude trifluoroacetic acid salt of the free acid of the title compound, 0.8g. This material was dissolved in water/acetonitrile, and the pH was adjusted to 6 with sodium bicarbonate. After freeze drying the formed solution, 0.9g crude title compound were obtained. Purification by column chromatography. M.P. = 274 °C.

## Example 70

**(S)-6-[[[3-[[[[2-Oxo-3-[[(phenylmethoxy)carbonyl]amino]-1-azetidinyl]carbonyl]amino]sulfonyl]-2-oxo-1-imidazolidinyl]amino]carbonyl]-3,4-bis(phenylmethoxy)-2-pyridinecarboxylic acid, 1,1-dimethyl-ethyl ester, monosodium salt**

Solution A: 2g of the title compound of Example 33, 0.7g N-hydroxybenzotriazole and 0.95g dicyclohexylcarbodiimide and 0.01g 4-dimethylaminopyridine were stirred for 1 hour at room temperature in 50ml tetrahydrofuran. The formed dicyclohexylurea was filtered off and washed with 10ml tetrahydrofuran. The filtrate was solution A.

Solution B: 2.7g of the title compound of Example 53H and 5.1ml N-methyl-N-trimethylsilyl-trifluoroacetic acid amide were stirred in 30ml acetonitrile for 1 hour at room temperature. The solvent and formed trifluoroacetic acid trimethylsilylester were distilled off in vacuo. The residue dissolved in 30ml tetrahydrofuran was solution B.

Solution A and B were then stirred overnight. The solvent distilled off and the residue stirred with 100ml

ice water and two drops of acetic acid. A solid residue of crude free acid of the title compound was isolated by filtration. It was dissolved in 30ml acetonitrile filtered and the filtrate was adjusted to pH 6 with sodiumbicarbonate solution. Crystals of title compound were formed and isolated by filtration after standing in an ice bath for 1 hour. 3g of title compound after drying. M.P. = 168-170 °C.

Example 71

(S)-6-[[[3-[[[[3-[[(2-Amino-4-thiazolyl)[(2-hydroxy-1,1-dimethyl-2-oxoethoxy)imino]acetyl]amino]-2-oxo-1-azetidinyl]carbonyl]amino]sulfonyl]-2-oxo-1-imidazolidinyl]amino]carbonyl]-1,4-dihydro-3-hydroxy-4-oxo-2-pyridinecarboxylic acid, trisodium salt

To 2.94g of the title compound of Example 70 in 50ml dimethylformamide were added 2.5ml N-methyl-N-trimethylsilyl-trifluoroacetic acid amide and 1.5g palladium/carbon catalyst (10%) after hydrogenation at room temperature for one hour, the catalyst was filtered off and washed with 20ml dimethylformamide. To the filtrate was added 1.89g 2-Amino-α-[[2-(diphenylmethoxy)-1,1-dimethyl2-oxoethoxy]imino]-4-thiazolacetic acid, 1H-benzotriazol-1-yl ester and the solution was stirred for 20 hours at room temperature. The solvent was then distilled off in vacuo and the residue stirred with ice water at pH 3.5 (acetic acid). 3.0g of the crude diphenylmethyl ester of the free acid of the title compound were obtained after filtration and drying.

1.5g of the above crude material were suspended in 3ml anisole and at -10 °C 20ml trifluoroacetic acid were added dropwise. After stirring for 1 hour at -10 °C and 2 hours at 0 °C the solvents were distilled off in vacuo and the residue stirred with 30ml ether yielding 0.9g solid of free acid title compound as a trifluoroacetic acid salt.

This material was dissolved in 20ml acetonitrile/water (1:1) at pH 6 (sodium bicarbonate) and filtered. The filtrate was evaporated in vacuo and redissolved in 30ml water and freeze dried, yielding 0.9g crude title compound and $CF_3COONa$. Purification of this material on XAD-2 water as an eluent gave pure compound. M.P. = 256 °C dec.

Example 72

[3S(Z)]-2-[[[1(2-Amino-4-thiazolyl)-2-[[1-[[[[3-[[(6-cyano-1,4-dihydro-5-hydroxy-4-oxo-2-pyridinyl)carbonyl]amino]-2-oxo-1-imidazolidinyl]sulfonyl]amino]carbonyl]-2-oxo-3-azetidinyl]amino]-2-oxoethylidene]amino]oxy]-2-methylpropoanoic acid, disodium salt

0.25g of the title compound of Example 49, 0.22g HOBT, 0.017g 4-dimethylaminopyridine and 0.29g dicyclohexylcarbodiimide were stirred in 15ml dimethylformamide for 30 minutes after adding 1g [3S(Z)]-2-[[[1-(2-Amino-4-thiazolyl)-2-[[1-[[[[3-amino-2-oxo-1-imidazolidinyl]sulfonyl]amino]carbonyl]-2-oxo-3-azetidinyl]amino]-2-oxoethylidene]amino]oxy]-2-methylpropanoic acid, diphenylmethyl ester. Stirring was continued for 18 hours. The formed dicyclohexylurea was filtered off and the dimethylformamide distilled off in vacuo. The residue was stirred in 10ml ethylacetate. A precipitate containing ~20% of [3S(Z)]-2-[[[1-(2-Amino-4-thiazolyl)-2-[[1-[[[[3-[[(6- cyano-1,4-dihydro-5-hydroxy-4-oxo-2-pyridinyl)carbonyl]amino]-2-oxo-1-imidazolidinyl]sulfonyl]amino]carbonyl]-2-oxo-3-azetidinyl]amino]-2-oxoethylidene]amino]oxy]-2-methylpropanoic acid, diphenylmethylester was obtained after filtration.

The material [3S(Z)]-2-[[[1-(2-Amino-4-thiazolyl)2-[[1-[[[[3-[[(6-cyano-1,4-dihydro-5-hydroxy-4-oxo-2-pyridinyl)carbonyl]amino]-2-oxo-1-imidazolidinyl]sulfonyl]amino]carbonyl]-2-oxo-3-azetidinyl]amino]-2-oxoethylidene]amino]oxy]-2-methylpropanoic acid, diphenylmethylester was stirred in 20ml trifluoroacetic acid/anisole (3:1) for one hour at 0 °C. At -10 °C ether was added and a crude precipitate of the trifluoroacetic acid salt of the free acid of the title compound, 1.2g, was obtained. This was suspended in 20ml ice water and the pH was adjusted to 5.8 with sodium bicarbonate. After filtration the filtrate was freeze dried, 1.1g. This material was chromatographed on XAD-2, water and water/acetonitrile as eluents. Fractions 59-70 contained 0.08g of the title compound of Example 16, fractions 71-80 0.03g, fractions 54-58

0.03g (H.I. 63%). The three combined fractions were chromatographed again on XAD-2, water as eluent. Fractions 66-64 contained 0.05g of title compound. M.P. = >275° C dec.

Example 73

[1-[[[[3-[[[6-difluoromethyl-4,5-bis(phenylmethoxy)-2-pyridinyl]carbonyl]amino]-2-oxo-1-imidazolidinyl]sulfonyl]-amino]carbonyl]-2-oxo-3-azetidinyl]carbamic acid, phenylmethylester, monosodium salt

1.2g of the title compound of Example 39, 0.46 N-hydroxybenzotriazole and 0.62 dicyclohexylcarbodiimide were stirred in 30ml tetrahydrofuran for one hour. The formed dicyclohexylurea was filtered off and washed with 10ml tetrahydrofuran. The combined filtrates are solution A.

1.8g trifluoroacetic acid salt of the title compound of Example 53H and 3ml N-methyl-N-trimethylsilyl-trifluoroacetic acid amide in 30ml acetonitrile were stirred for one hour at room temperature. The solvent and formed trimethylsilyltrifluoroacetate were distilled off in vacuo and the residue redissolved in 30ml tetrahydrofuran. This is solution B.

Solutions A and B were stirred together overnight at room temperature. The solvent was distilled off then, and the residue stirred with 50ml ice water and 1 drop acetic acid for 1 hour. A white solid of crude [1-[[[[3-[[[6difluromethyl4,5-bis(phenylmethoxy)-2-pyridinyl]carbonyl]amino]2-oxo-1-imidazolidinyl]sulfonyl]-amino]carbonyl]-2oxo-3-azetidinyl]carbamic acid, phenylmethylester was formed, 2.3g.

3g crude [1-[[[[3-[[[6-difluromethyl-4,5-bis(phenylmethoxy)-2-pyridinyl]carbonyl]amino]-2-oxo-1-imidazolidinyl]sulfonyl]amino]carbonyl]-2-oxo-3-azetidinyl]carbamic acid, phenylmethylester were dissolved in acetonitrile and filtered (0.3g residue). To the filtrate was added sodiumbicarbonate solution (water) until the pH showed 6.5. After adding a little more water, crystals of the title compound were formed. They were isolated after standing in the cold for several hours. Yield: 1.7g title compound. M.P. = 170° C dec.

Example 74

(S)-[1-[[[[3-[[[6-(2-cyanoethenyl)-4,5-bis (phenylmethoxy)-2-pyridinyl]carbonyl]amino]-2-oxo-1-imidazolidinyl]-sulfonyl]amino]carbonyl]-2-oxo-3-azetidinyl]carbamic acid, phenylmethyl ester

Solution A: 4.65g trifluoroacetic acid salt of the title compound of Example 53H and 8ml N-methyl-N-trimethylsilyl-trifluoroacetic acid amide were stirred in 50ml acetonitrile for one hour at room temperature. The solvent and formed trimethylsilyltrifluoroacetate were distilled off in vacuo then. The residue was dissolved in 50ml tetrahydrofuran.

Solution B: To 3g of the title compound of Example 40, dissolved in 30ml tetrahydrofuran and 10ml dimethylformamide, were added 1.19g HOBT, 1.61g dicyclohexylcarbodiimide and 0.01g 4-dimethylaminopyridine. After stirring for 2-1/2 hours at room temperature, the formed dicyclohexylurea was filtered off and washed with 5ml dimethylformamide.

Solutions A and B were stirred together at room temperature overnight. The solvents were distilled off and the residue stirred in ice water at pH 3.5 (acetic acid) for one hour. The precipitate of crude title compound was filtered off and dissolved in acetonitrile. The pH was adjusted to 6.0 with sodium bicarbonate solution. After adding water, crystals of the sodium salt of the title compound were formed. They were isolated by filtration and dried. Yield: 4.1g. After suspension in ethyl acetate/water the pH was adjusted to 3.5 with citric acid. The organic phase gave 4.0g of title compound after washing with water and drying. M.P. = 140° C dec.

Example 75

[3S(Z)]-2-[[[1-(2-Amino-4-thiazolyl)-2-[[1-[[[[3-[[(6-formyl-1,4-dihydro-5-hydroxy-4-oxo-2-pyridinyl)carbonyl]amino]-2-oxo-1-imidazolidinyl]sulfonyl]amino]carbonyl]-4-oxo-3-azetidinyl]amino]-2-oxoethylidene]amino]oxy]-2-methylpropanoic acid, disodium salt

1.63g of the title compound of Example 73 and 1.5ml N-methyl-N-trimethylsilyl-trifluoroacetic acid amide were dissolved in 30ml dimethylformamide. 0.8g palladium/carbon (10%) were added. With stirring a stream of hydrogen was bubbled through the reaction suspension for 1 hour. The catalyst was filtered off then. To the filtrate was added 1.11g 2-Amino-(α-[[2-(diphenylmethoxy)-1,1-dimethyl-2-oxoethoxy]imino]-4-thiazolacetic acid, 1H-benzotriazol-1-yl ester. After stirring overnight, the solvent was distilled off in vacuo and the residue stirred for one hour with ice water containing 1 drop of acetic acid. A precipitate of crude [3S(Z)]-2-[[[1-(2-amino-4-thiazolyl)-2-[[1-[[[[3-[[[(difluoromethyl)-1,4-dihydro-5-hydroxy-4-oxo-2-pyridinyl]-carbonyl]amino]-2-oxo-1-imidazolidinyl]sulfonyl]amino]carbonyl]-2-oxo-3-azetidinyl]amino]2-oxoethylidene]-amino]oxy]-2-methylpropanoic acid, diphenylmethyl ester, 1.7g, was obtained after drying. 1.7g [3S(Z)]-2-[[-[1-(2-Amino-4-thiazolyl)-2-[[1-[[[[3-[[[(difluoromethyl)-1,4-dihydro-5-hydroxy-4-oxo-2-pyridinyl]carbonyl]-amino]-2-oxo-1-imidazolidinyl]sulfonyl]amino]carbonyl]-2-oxo-3-azetidinyl]-amino]-2-oxoethylidene]amino]-oxy]-2-methylpropanoic acid, diphenylmethyl ester, (crude) were suspended in 5ml anisole at -10 °C and 30ml trifluoroacetic acid were added dropwise during a period of 30 minutes. After 1 hour 100ml ether were added. A solid trifluoroacetic acid salt of an unknown structure was obtained, 1.69g. This material was dissolved in acetonitrile, and the pH was adjusted to 6.0 with sodium bicarbonate solution (water). The color turned yellow. After filtration the acetonitrile was distilled off in vacuo and the residual water solution freeze dried. Yield: 1.9g crude title compound and purified by column chromatography. M.P. = 252 °C dec.

## Claims

1. Compounds having the formula

I

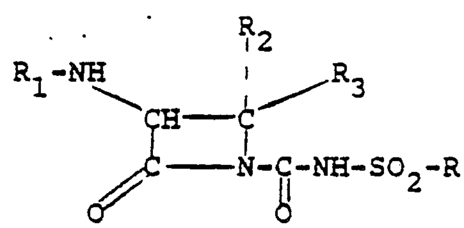

, (I)

and pharmaceutically acceptable salts thereof, wherein

R is 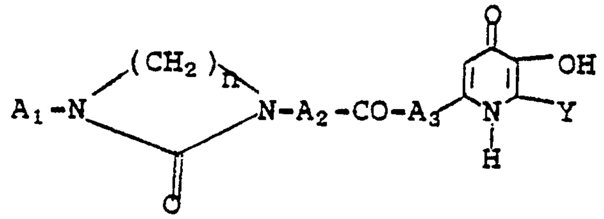 ;

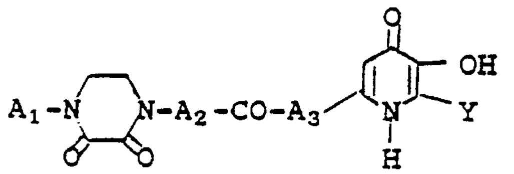 ; 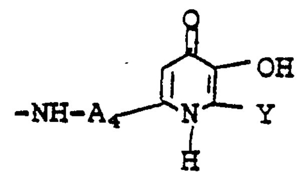 ;

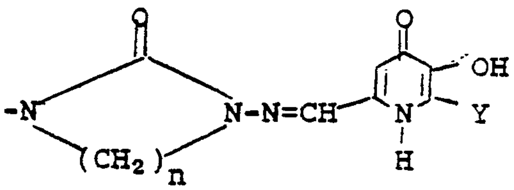 or 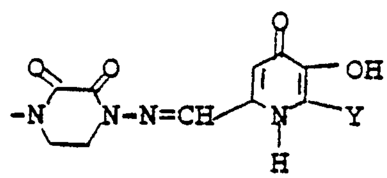

$R_1$ is an acyl group derived from a carboxylic acid;
$R_2$ and $R_3$ are the same or different and each is hydrogen, alkyl, alkenyl, alkynyl, cycloalkyl, phenyl, substitited phenyl or a 4, 5, 6 or 7-membered herterocycle (hereinafter referred to as $R_x$), or one of $R_2$ and $R_3$ is hydrogen and the other is azido, halomethyl, dihalomethyl, trihalomethyl, alkoxycarbonyl, 2-phenylethenyl, 2-phenylethynyl, carboxyl, $-CH_2X_1$ [wherein $X_1$ is azido, amino ($-NH_2$), hydroxy, carboxyl, alkoxycarbonyl, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano, $-A-\overset{O}{\overset{\|}{C}}-NX_6X_7$, $S-X_2$, or $-O-X_2$ (wherein A, $X_2$, $X_6$ and $X_7$ are as hereinafter defined)], $-S-X_2$ or $-O-X_2$ - [wherein $X_2$ is alkyl, substituted alkyl, phenyl, substituted phenyl, phenylalkyl, (substituted phenyl)alkyl, alkanoyl, phenylalkanoyl, (substituted phenyl)alkanoyl, phenylcarbonyl, (substituted phenyl)carbonyl, or heteroarylcarbonyl],

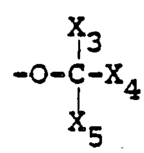 or

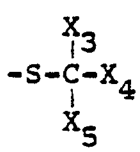

[wherein one of $X_3$ and $X_4$ is hydrogen and the other is hydrogen or alkyl, or $X_3$ and $X_4$ when taken together with the carbon atom to which they are attached form a cycloalkyl group; and $X_5$ is formyl, alkanoyl, substituted alkanoyl, phenylcarbonyl, (substituted phenyl)carbonyl, phenylalkylcarbonyl, (substituted phenyl)alkylcarbonyl, carboxyl, alkoxycarbonyl, aminocarbonyl ($NH_2-\overset{O}{\overset{\|}{C}}-$), (substituted amino)carbonyl, or cyano ($-C\equiv N$)], or $-A-\overset{O}{\overset{\|}{C}}-NX_6X_7$ [wherein A is $-CH=CH-$, $-(CH_2)_m-$, $-(CH_2)_m-O-$, $-(CH_2)_m-NH-$, or $-CH_2-S-CH_2-$, m is 0, 1 or 2, and $X_6$ and $X_7$ are the same or different and each is hydrogen, alkyl, phenyl or substituted phenyl, or $X_6$ is hydrogen and $X_7$ is amino, substituted amino, alkanoylamino or alkoxy, or $X_6$ and $X_7$ when taken together with the nitrogen atom to which they are attached form a 4, 5, 6 or 7-membered heterocycle];

$A_1$ is a single bond, NH or 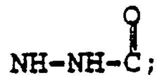;

$A_2$ is a single bond, NH, $CH_2$-$CH_2$-NH or

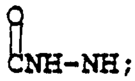;

$A_3$ is a single bond, CH=CH, $(CH_2)_2$-;

$A_4$ is 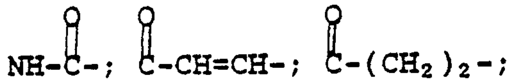;

n is two or three

Y is $CH_2X$, $CHF_2$, 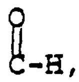, C≡N, 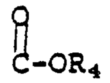, 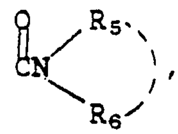,

$OR_7$, $CH=NR_8$, $CH=CHR_9$, 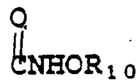, or 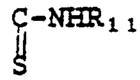

X is hydrogen, halogen, OH, $OR_{12}$, $NH_2$,

$NHR_{13}$, $N_3$, 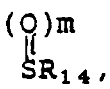, C≡N, S-C≡N, 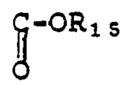, or

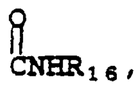, 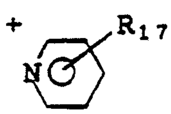

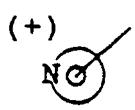 (6 or 5 membered heterocycles containing one or two nitrogen atoms)

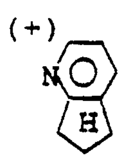 ; 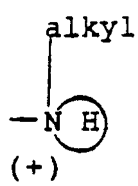 (saturated 5 or 6 membered)

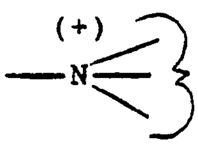 lower alkyl; 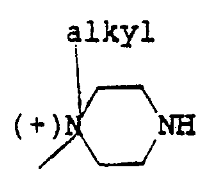

$R_4$ is hydrogen, salt forming metal or ion, lower alkyl;
$R_5$ and $R_6$ is hydrogen or lower alkyl or joined together to form a five or six membered ring;
$R_7$ is hydrogen, lower alkyl, aralkyl;
$R_8$ is O-lower alkyl, hydroxy, NH-alkyl, NH-aryl;
$R_9$ is C≡N, COOH, COO-lower alkyl, $CONH_2$, O-lower alkyl, S-lower alkyl;

$R_{10}$ is hydrogen, lower alkyl;
$R_{11}$ is hydrogen, lower alkyl;
$R_{12}$ is hydrogen, lower alkyl, aralkyl,

$\overset{O}{\overset{\|}{C}}NH_2$, $\overset{O}{\overset{\|}{C}}NH$-lower alkyl, $\overset{O}{\overset{\|}{C}}NH$-aryl;

$R_{13}$ is lower alkyl, $\overset{O}{\overset{\|}{C}}NH_2$, $\overset{O}{\overset{\|}{C}}NH$-lower alkyl,

$\overset{O}{\overset{\|}{C}}NH$-aryl, $\overset{O}{\overset{\|}{C}}NH$-heterocycle, $\underset{S}{\underset{\|}{C}}NH_2$, $\underset{S}{\underset{\|}{C}}NH$-lower alkyl,

$\underset{S}{\underset{\|}{C}}NH$-aryl;

$R_{14}$ is lower alkyl, m is zero, one, or two;
$R_{15}$ is hydrogen, salt forming metal ion, lower alkyl;
$R_{16}$ is hydrogen, lower alkyl;
$R_{17}$ is hydrogen, $CONH_2$, COOH.

2. A compound according to claim 1 wherein R is

R is 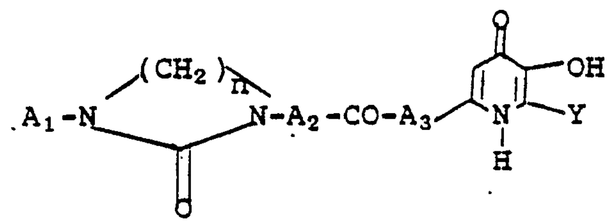

3. A compound according to claim 1 wherein R is

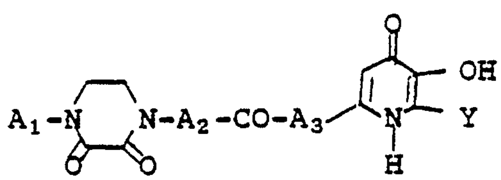

4. A compound according to claim 1 wherein R is

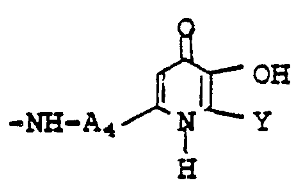

5. A compound according to claim 1 wherein R is

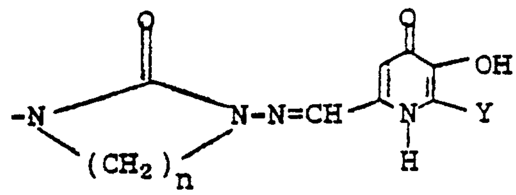

6. A compound according to claim 1 wherein R is

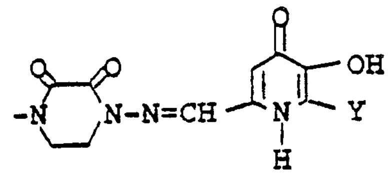

7. A compound according to any of claims 1 to 6 wherein $R_2$ and $R_3$ are the same or different and each is hydrogen or alkyl.

8. A compound according to any of claims 1 to 7 wherein $R_1$ is

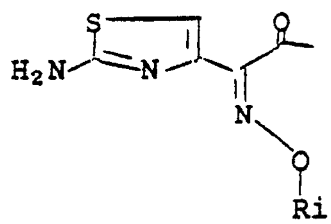

where Ri is hydrogen, alkyl, cycloalkyl, carboxymethyl, 1-carboxy-1-ethyl (R and S isomers), 1-carboxy-1-methyethyl, 2,2,2-trifluoroethyl, 1-carboxycyclopropyl, 1-carboxycyclobutyl, or 1-carboxycyclopentyl.

9. A compound of claim 2 wherein $R_2$ and $R_3$ are the same or different and each is hydrogen or alkyl.

10. A compound of claim 2 wherein Ri is

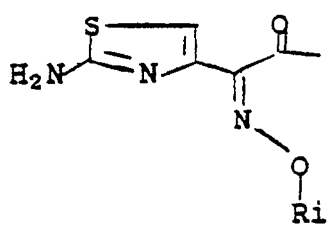

where Ri is hydrogen, alkyl, cycloalkyl, carboxymethyl, 1-carboxy-1-ethyl (R and S isomers), 1-carboxy-1-methyethyl, 2,2,2-trifluoroethyl, 1-carboxycyclopropyl, 1-carboxycyclobutyl, or 1-carboxycyclopentyl.

11. A compound of claim 10 wherein $R_2$ and $R_3$ are the same or different and each is hydrogen or alkyl.

12. A compound according to claim 1, [3S(Z)]-2-[[[1-(2-Amino-4-thiazolyl)-2-[[1-[[[[3-[[(1,4-dihydro-5-hydroxy-6-methyl-4-oxo-2-pyridinyl)carbonyl]amino]-2-oxo-1-imidazolidinyl]sulfonyl]amino]carbonyl]-2-oxo-3-azetidinyl]amino]-2-oxoethylidene]amino]oxy]-2-methylpropanoic acid, disodium salt.

13. A compound according to claim 1, [3S(Z)]-2-[[[1-(2-Amino-4-thiazolyl)-2-[[1-[[[[3-[[(1,4-dihydro-5-hydroxy-6-methoxy-4-oxo-2-pyridinyl)carbonyl]amino]-2-oxo-1-imidazolidinyl]sulfonyl]amino]carbonyl]-2-oxo-3-azetidinyl]amino]-2-oxoethylidene]-amino]oxy]-2-methyl-propanoic acid, disodium salt.

14. A compound according to claim 1, [3S(Z)]-2-[[[1-(2-Amino-4-thiazolyl)-2-[[1-[[[[3-[[[1,4-dihydro-5-hydroxy-4-oxo-6-(pyridiniomethyl)-2-pyridinyl]carbonyl]amino]-2-oxo-1-imidazolidinyl]sulfonyl]amino]-carbonyl]-2-oxo-3-azetidinyl]amino-2-oxoethylidene]-amino]oxy]-2-methylpropanoic acid, monosodium salt.

15. A compound according to claim 1, [3S(Z)]-2-[[[1-(2-Amino-4-thiazolyl)-2-[[1-[[[[3-[[[1,4-dihydro-5-hydroxy-4-oxo-6-(pyridiniomethyl)-2-pyridinyl]carbonyl]amino]-2-oxo-1-imidazolidinyl]sulfonyl]amino]-carbonyl]-2-oxo-3-azetidinyl]amino]-2-oxoethylidene]-amino]oxy]-2-methylpropanoic acid, monosodium salt.

16. A compound according to claim 1, [3S(Z)]-2-[[[1-(2-Amino-4-thiazolyl)-2-[[1-[[[[3-[[[1,4-dihydro-5-hydroxy-6-(hydroxy-methyl)-4-oxo-2-pyridinyl]carbonyl]amino]-2-oxo-1-imidazolidinyl]sulfonyl]amino]-carbonyl]-2-oxo-3-azetidinyl]amino]-2-oxoethylidene]-amino]oxy]propanoic acid, disodium salt.

17. A compound according to claim 1, 2-[[[1-(2-Amino-4-thiazolyl)-2-[[1-[[[[3-[[(1,4-dihydro-5,6-dihydroxy-4-oxo-2-pyridinyl]carbonyl]amino]-2-oxo-1-imidazolidinyl]sulfonyl]amino]carbonyl]-2-oxo-3-azetidinyl]amino]-2-oxo-ethylidene]amino]oxy]-2-methylpropanoic acid, disodium salt.

18. A compound according to claim 1, [3S(Z)]-2-[[[2-[[1-[[[[3-[[[6-(Aminocarbonyl)-1,4-dihydro-5-hydroxy-4-oxo-2-pyridinyl)carbonyl]amino]-2-oxo-1-imidazolidinyl]sulfonyl]amino]carbonyl-2-oxo-3-azetidinyl]-amino]-1-(2-amino-4-thiazolyl)-2-oxoethylidene]-amino]oxy]-2-methylpropanoic acid, disodium salt.

19. A compound according to claim 1, (S)-6-[[[3-[[[[3-[[(2-Amino-4-thiazolyl)[(2-hydroxy-1,1-dimethyl-2-oxoethoxy)imino]acetyl]amino]-2-oxo-1-azetidinyl]carbonyl]amino]sulfonyl]-2-oxo-1-imidazolidinyl]amino]-carbonyl]-1,4-dihydro-3-hydroxy-4-oxo-2-pyridinecarboxylic acid, trisodium salt.

20. A compound according to claim 1, [3S(Z)]-2-[[[1-(2-Amino-4-thiazolyl)-2-[[1-[[[[3-[[(6-cyano-1,4-dihydro-5-hydroxy-4-oxo-2-pyridinyl)carbonyl]amino]-2-oxo-1-imidazolidinyl]sulfonyl]amino]carbonyl]-2-oxo-3-azetidinyl]amino]-2-oxoethylidene]amino]oxy]-2-methylpropoanoic acid, disodium salt.

21. A compound according to claim 1, [3S(Z)]-2-[[[1-(2-Amino-4-thiazolyl)-2-[[1-[[[[3-[[(6-formyl-1,4-dihydro-5-hydroxy-4-oxo-2-pyridinyl)carbonyl]amino]-2-oxo-1-imidazolidinyl]sulfonyl]amino]carbonyl]-4-oxo-3-azetidinyl]amino]-2-oxoethylidene]-amino]oxy]-2-methylpropanoic acid, disodium salt.

22. Compounds represented by the formula

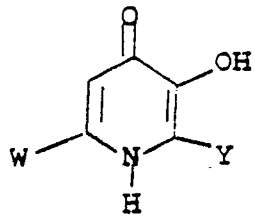

(XXIX a)

and

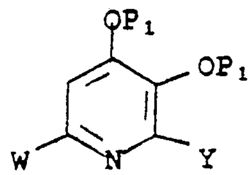

(XXIX)

wherein W is 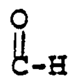 , COOH or -CH=CHCOOH

Y is $CH_2X$, $CHF_2$, 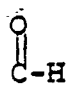, C≡N, 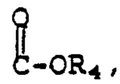, 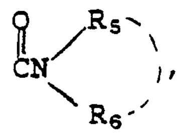,

$OR_7$, $CH=NR_8$, $CH=CHR_9$, 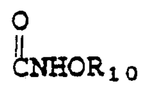, or 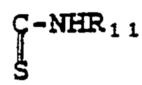

X is hydrogen, halogen, OH, $OR_{12}$, $NH_2$,

$NHR_{13}$, $N_3$, 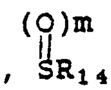, C≡N, S-C≡N, 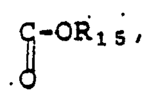, or

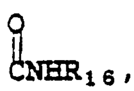, 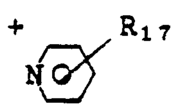

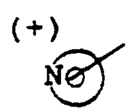 (6 or 5 membered heterocycles containing one or two nitrogen atoms)

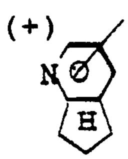 ; 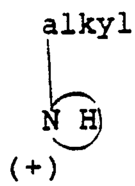 (saturated 5 or 6 membered)

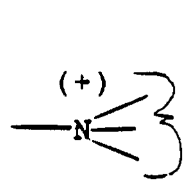 lower alkyl; 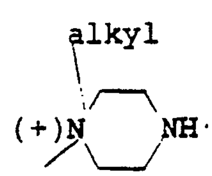

$R_4$ is hydrogen, salt forming metal or ion, lower alkyl;

$R_5$ and $R_6$ is hydrogen or lower alkyl or joined together to form a five or six membered ring;

$R_7$ is hydrogen, lower alkyl, aralkyl;
$R_8$ is O-lower alkyl, hydroxy, NH-alkyl, NH-aryl;
$R_9$ is C≡N, COOH, COO-lower alkyl, $CONH_2$, O-lower alkyl, S-lower alkyl;
$R_{10}$ is hydrogen, lower alkyl;
$R_{11}$ is hydrogen, lower alkyl;
$R_{12}$ is hydrogen, lower alkyl, aralkyl,

$\overset{O}{\overset{\|}{C}}NH_2$, $\overset{O}{\overset{\|}{C}}$NH-lower alkyl, $\overset{O}{\overset{\|}{C}}$NH-aryl;

$R_{13}$ is lower alkyl, $\overset{O}{\overset{\|}{C}}NH_2$, $\overset{O}{\overset{\|}{C}}$NH-lower alkyl,

$\overset{O}{\overset{\|}{C}}$NH-aryl, $\overset{O}{\overset{\|}{C}}$NH-heterocycle, $\underset{S}{\underset{\|}{C}}NH_2$, $\underset{S}{\underset{\|}{C}}$NH-lower alkyl,

$\underset{S}{\underset{\|}{C}}$NH-aryl;

$R_{14}$ is lower alkyl, m is zero, one, or two;
$R_{15}$ is hydrogen, salt forming metal ion, lower alkyl;
$R_{16}$ is hydrogen, lower alkyl;
$R_{17}$ is hydrogen, $CONH_2$, COOH, and $P_1$ is hydrogen or a hydroxyl protecting group.

23. A compound according to claim 22 wherein the hydroxyl protecting group is benzyl alkyloxycarbonyl, alkanoyl, phenyl carbonyl or substituted phenyl carbonyl.

24. A compound according to claim 22 wherein W is COOH.

25. A compound according to claim 22, 6-Methyl-4,5-bis(phenylmethoxy)-2-pyridinecarboxylic acid.

26. A compound according to claim 22, 6-(chloromethyl)-4,5-bis(phenylmethoxy)-2-pyridinecarboxylic acid, methyl ester.

27. A compound according to claim 22, 6-(Azidomethyl)-4,5-bis(phenylmethoxy)-2-pyridinecarboxylic acid.

28. A compound according to claim 22, 6-(Aminomethyl)-4,5-bis(phenylmethoxy)-2-pyridinecarboxylic acid.

29. A compound according to claim 22, 6-[[[(1,1-Dimethylethoxy)carbonyl]amino]-methyl]-4,5-bis-(phenylmethoxy)-2-pyridinecarboxylic acid.

30. A compound according to claim 22, 6-[[(Aminocarbonyl)amino]methyl]-4,5-bis(phenylmethoxy)-2-pyridine-carboxylic acid.

31. A compound according to claim 22, 3,4-Bis(phenylmethoxy)-2,6-pyridinedicarboxylic acid, 6-(phenylmethyl) ester.

32. A compound according to claim 22, 3,4-Bis(phenylmethoxy)-2,6-pyridine-carboxylic acid, 2-(1,1-dimethylethyl) ester.

33. A compound according to claim 22, 6-Cyano-4,5-bis(phenylmethoxy)-2-pyridinecarboxylic acid.

34. A compound according to claim 22, 6-(Aminocarbonyl)-4,5-bis(phenylmethoxy)-2-pyridinecarboxylic acid.

35. A compound according to claim 22, 6-difluoromethyl-4,5-bis(phenylmethoxy)-2-pyridinecarboxylic acid.

36. A compound according to claim 22, 6-Methoxy-4,5-bis(phenylmethoxy)-2-pyridinecarboxylic acid, phenylmethyl ester.

37. A compound according to claim 22, 1,6-Dihydro-6-oxo-4,5-bis(phenylmethoxy)-2-pyridinecarboxylic acid.

38. A compound according to claim 22, 4,5,6-Tris(phenylmethoxy)-2-pyridinecarboxylic acid.

39. A compound according to claim 22, 6-Cyano-4,5-dihydroxy-2-pyridinecarboxylic acid.

40. A compound having the name 6-(Chloromethyl)-4,5-bis(phenylmethoxy)-2-pyridinecarbonylchloride Hydrochloride

41. Pharmaceutical composition comprising a pharmaceutically effective amount of a compound according to any of claims 1 to 21 or its pharmaceutically acceptable salt.

42. Pharmaceutical compositions for combatting bacterial infections in mammals comprising a pharmaceutically effective amount of a compound according to any of claims 1 to 21 or its pharmaceutically acceptable salt.

43. Use of the compounds according to any of claims 1 to 21 or of their pharmaceutically acceptable salts for the preparation of a composition according to claim 42.

European Patent Office

# EUROPEAN SEARCH REPORT

Application number: EP 89105883.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A | US - A - 4 743 685 (BREUER) * Abstract; formulas XXIII, XXV; claim 1 * | 1,22, 41-43 | C 07 D 401/14<br>C 07 D 401/06<br>C 07 D 213/69<br>A 61 K 31/44<br>A 61 K 31/495<br>A 61 K 31/505 |
| P,A | US - A - 4 772 693 (BREUER) * Claim 1; column 9, lines 40-59 * | 1,41-43 | |
| A | EP - A1 - 0 246 786 (UPJOHN) * Abstract; formulas I,III * | 1,41-43 | |
| A | EP - A1 - 0 204 207 (SQUIBB) * Abstract * | 1,41-43 | |
| A | EP - A3 - 0 243 924 (SQUIBB) * Abstract * | 1,41-43 | |

TECHNICAL FIELDS SEARCHED (Int. Cl.4)

C 07 D 401/00
C 07 D 213/00

The present search report has been drawn up for all claims

| Place of search | Date of completion of the search | Examiner |
|---|---|---|
| VIENNA | 14-06-1989 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82